# EUROPEAN PATENT SPECIFICATION

(11) **EP 1 968 565 B1**
(45) Date of publication and mention of the grant of the patent: **18.11.2015**
(21) Application number: 05784880.6
(22) Date of filing: 18.04.2005
(51) Int. Cl.: C07K 14/52

(54) **COMPOSITIONS AND METHODS FOR INHIBITING ANGIOGENESIS**
ZUSAMMENSETZUNGEN UND VERFAHREN ZUR UNTERDRÜCKUNG DER ANGIOGENESE
COMPOSITIONS ET PROCEDES PERMETTANT D'INHIBER L'ANGIOGENESE

(30) Priority: 16.04.2004 US 562841 P
(43) Date of publication of application: 17.09.2008
(73) Proprietor: Sun, Baocun, Tianjin 300060 (CN); Cao, Yihai, 168 59 Bromma (SE)
(72) Inventor: Cao, Yihai, 168 59 Bromma (SE); Baocun, Sun, Tianjin 300060 (CN)
(74) Representative: Brann AB
(86) International application number: PCT/US2005/013079
(87) International publication number: WO 2006/001888

(56) References cited:
- BJORNDAHL MEIT ET AL: "Blockage of VEGF- induced angiogenesis by preventing VEGF secretion" CIRCULATION RESEARCH, vol. 94, no. 11, 11 June 2004 (2004-06-11), pages 1443-1450, XP002553705 ISSN: 0009-7330
- JOHANSEN T E ET AL: "C-terminal KDEL-modified cystatin C is retained in transfected CHO cells" BIOCHEMICAL AND BIOPHYSICAL RESEARCH COMMUNICATIONS, ACADEMIC PRESS INC. ORLANDO, FL, US, vol. 172, no. 3, 15 November 1990 (1990-11-15), pages 1384-1391, XP024843804 ISSN: 0006-291X [retrieved on 1990-11-15]
- ERIKSSON A ET AL: "Placenta growth-factor-1 antagonizes VEGF-induced angiogenesis and tumor growth by the formation of functionally inactive P1GF-1/VEGF heterodimers" CANCER CELL, CELL PRESS, US, vol. 1, 1 February 2002 (2002-02-01), pages 99-108, XP002967596 ISSN: 1535-6108
- VERHEUL H M W ET AL: "VASCULAR ENDOTHELIAL GROWTH FACTOR AND ITS INHIBITORS" DRUGS OF TODAY / MEDICAMENTOS DE ACTUALIDAD, J.R. PROUS SS.A. INTERNATIONAL PUBLISHERS, ES, vol. 39, no. SUPPL. C, 1 January 2003 (2003-01-01), pages 81-93, XP008033177 ISSN: 0025-7656
- SHINKARUK S ET AL: "Vascular Endothelial Cell Growth Factor (VEGF), an emerging target for cancer chemotherapy" CURRENT MEDICINAL CHEMISTRY. ANTI-CANCER AGENTS, BENTHAM SCIENCE PUBLISHERS, HILVERSUM, NL, vol. 3, no. 2, 1 March 2003 (2003-03-01), pages 95-117, XP009125135 ISSN: 1568-0118
- OZAWA ET AL.: 'Expression of the oxygen-regulated protein ORP150 accelerates wound Healing by modulating intracellular VEGF transport.' JOURNAL OF CLINICAL INVESTIGATION vol. 108, no. 1, July 2001,

### SEQUENCE LISTING

<110> ACUITY PHARMACEUTICALS, INC. Cao, Yihai
<120> Compositions and Methods for Inhibiting Angiogenesis
<130> 129402.00602
<140> not yet assigned
   <141> 2005-04-18
<150> 60/562,841 <151> 2004-04-16
<160> 24
<170> PatentIn version 3.2
<210> 1
   <211> 451
   <212> DNA
   <213> Homo sapiens
<220>
   <223> PLGF-1 Nucleotide sequence for cDNA
<400> 1
   gatgccggtc atgaggctgt tcccttgctt cctgcagctc ctggccgggc tggcgctgcc      60
   tgctgtgccc ccccagcagt gggccttgtc tgctgggaac ggctcgtcag aggtggaagt     120
   ggtacccttc caggaagtgt ggggccgcag ctactgccgg gcgctggaga ggctggtgga     180
   cgtcgtgtcc gagtacccca gcgaggtgga gcacatgttc agcccatcct gtgtctccct     240
   gctgcgctgc accggctgct gcggcgatga gaatctgcac tgtgtgccgg tggagacggc     300
   caatgtcacc atgcagctcc taaagatccg ttctggggac cggccctcct acgtggagct     360
   gacgttctct cagcacgttc gctgcgaatg ccggcctctg cgggagaaga tgaagccgga     420
   aaggtgcggc gatgctgttc cccggaggta a                                    451
<210> 2
   <211> 149
   <212> PRT
   <213> Artificial
<220>
   <223> PLGF-1 amino acid sequence without KDEL
<400> 2
   Met Pro Val Met Arg Leu Phe Pro Cys Phe Leu Gln Leu Leu Ala Gly
   1 5 10 15
   Leu Ala Leu Pro Ala Val Pro Pro Gln Gln Trp Ala Leu Ser Ala Gly
   20 25 30
   Asn Gly Ser Ser Glu Val Glu Val Val Pro Phe Gln Glu Val Trp Gly
   35 40 45
   Arg Ser Tyr Cys Arg Ala Leu Glu Arg Leu Val Asp Val Val Ser Glu
   50 55 60
   Tyr Pro Ser Glu Val Glu His Met Phe Ser Pro Ser Cys Val Ser Leu
   65 70 75 80
   Leu Arg Cys Thr Gly Cys Cys Gly Asp Glu Asn Leu His Cys Val Pro
   85 90 95
   Val Glu Thr Ala Asn Val Thr Met Gln Leu Leu Lys Ile Arg Ser Gly
   100 105 110
   Asp Arg Pro Ser Tyr Val Glu Leu Thr Phe Ser Gln His Val Arg Cys
   115 120 125
   Glu Cys Arg Pro Leu Arg Glu Lys Met Lys Pro Glu Arg Cys Gly Asp
   130 135 140
   Ala Val Pro Arg Arg
   145
<210> 3
   <211> 153
   <212> PRT
   <213> Artificial
<220>
   <223> PLGF-1 amino acid sequence with KDEL
<400> 3
   Met Pro Val Met Arg Leu Phe Pro Cys Phe Leu Gln Leu Leu Ala Gly
   1               5                   10                  15
   Leu Ala Leu Pro Ala Val Pro Pro Gln Gln Trp Ala Leu Ser Ala Gly
               20                  25                  30
   Asn Gly Ser Ser Glu Val Glu Val Val Pro Phe Gln Glu Val Trp Gly
           35                  40                  45
   Arg Ser Tyr Cys Arg Ala Leu Glu Arg Leu Val Asp Val Val Ser Glu
       50                  55                  60
   Tyr Pro Ser Glu Val Glu His Met Phe Ser Pro Ser Cys Val Ser Leu
   65                  70                  75                  80
   Leu Arg Cys Thr Gly Cys Cys Gly Asp Glu Asn Leu His Cys Val Pro
                   85                  90                  95
   Val Glu Thr Ala Asn Val Thr Met Gln Leu Leu Lys Ile Arg Ser Gly
               100                 105                 110
   Asp Arg Pro Ser Tyr Val Glu Leu Thr Phe Ser Gln His Val Arg Cys
           115                 120                 125
   Glu Cys Arg Pro Leu Arg Glu Lys Met Lys Pro Glu Arg Cys Gly Asp
       130                 135                 140
   Ala Val Pro Arg Arg Lys Asp Glu Leu
   145                 150
<400> 4
<210> 5
   <211> 170
   <212> PRT
   <213> Artificial
<220>
   <223> PLGF-2 amino acid sequence without KDEL
<400> 5
   Met Pro Val Met Arg Leu Phe Pro Cys Phe Leu Gln Leu Leu Ala Gly
   1 5 10 15
   Leu Ala Leu Pro Ala Val Pro Pro Gln Gln Trp Ala Leu Ser Ala Gly
   20 25 30
   Asn Gly Ser Ser Glu Val Glu Val Val Pro Phe Gln Glu Val Trp Gly
   35 40 45
   Arg Ser Tyr Cys Arg Ala Leu Glu Arg Leu Val Asp Val Val Ser Glu
   50 55 60
   Tyr Pro Ser Glu Val Glu His Met Phe Ser Pro Ser Cys Val Ser Leu
   65 70 75 80
   Leu Arg Cys Thr Gly Cys Cys Gly Asp Glu Asn Leu His Cys Val Pro
   85 90 95
   Val Glu Thr Ala Asn Val Thr Met Gln Leu Leu Lys Ile Arg Ser Gly
   100 105 110
   Asp Arg Pro Ser Tyr Val Glu Leu Thr Phe Ser Gln His Val Arg Cys
   115 120 125
   Glu Cys Arg Pro Leu Arg Glu Lys Met Lys Pro Glu Arg Arg Arg Pro
   130 135 140
   Lys Gly Arg Gly Lys Arg Arg Arg Glu Lys Gln Arg Pro Thr Asp Cys
   145 150 155 160
   His Leu Cys Gly Asp Ala Val Pro Arg Arg
   165 170
<210> 6
   <211> 174
   <212> PRT
   <213> Artificial
<220>
   <223> PLGF-1 amino acid with KDEL
<400> 6
   Met Pro Val Met Arg Leu Phe Pro Cys Phe Leu Gln Leu Leu Ala Gly
   1 5 10 15
   Leu Ala Leu Pro Ala Val Pro Pro Gln Gln Trp Ala Leu Ser Ala Gly
   20 25 30
   Asn Gly Ser Ser Glu Val Glu Val Val Pro Phe Gln Glu Val Trp Gly
   35 40 45
   Arg Ser Tyr Cys Arg Ala Leu Glu Arg Leu Val Asp Val Val Ser Glu
   50 55 60
   Tyr Pro Ser Glu Val Glu His Met Phe Ser Pro Ser Cys Val Ser Leu
   65 70 75 80
   Leu Arg Cys Thr Gly Cys Cys Gly Asp Glu Asn Leu His Cys Val Pro
   85 90 95
   Val Glu Thr Ala Asn Val Thr Met Gln Leu Leu Lys Ile Arg Ser Gly
   100 105 110
   Asp Arg Pro Ser Tyr Val Glu Leu Thr Phe Ser Gln His Val Arg Cys
   115 120 125
   Glu Cys Arg Pro Leu Arg Glu Lys Met Lys Pro Glu Arg Arg Arg Pro
   130 135 140
   Lys Gly Arg Gly Lys Arg Arg Arg Glu Lys Gln Arg Pro Thr Asp Cys
   145 150 155 160
   His Leu Cys Gly Asp Ala Val Pro Arg Arg Lys Asp Glu Leu
   165 170
<210> 7
   <211> 4
   <212> PRT
   <213> Artificial
<220>
   <223> Endoplasmic reticulum retention signal for a protein
<400> 7
   Lys Asp Glu Leu
   1
<210> 8
   <211> 12
   <212> DNA
   <213> Homo sapiens
<220>
   <223> nt sequence for KDEL
<400> 8
   aargayggrt tr 12
<210> 9
   <211> 12
   <212> DNA
   <213> Homo sapiens
<220>
   <223> nt sequence for KDEL
<220>
   <221> misc_feature
   <222> (12)..(12)
   <223> n is a, c, g, or t
<400> 9
   aargayggrc tn 12
<210> 10
   <211> 624
   <212> DNA
   <213> Homo sapiens
<220>
   <223> VEGF-B isoform 1 nucleotide sequence for cDNA
<400> 10
   atgagccctc tgctccgccg cctgctgctc gccgcactcc tgcagctggc ccccgcccag 60
   gcccctgtct cccagcctga tgcccctggc caccagagga aagtggtgtc atggatagat 120
   gtgtatactc gcgctacctg ccagccccgg gaggtggtgg tgcccttgac tgtggagctc 180
   atgggcaccg tggccaaaca gctggtgccc agctgcgtga ctgtgcagcg ctgtggtggc 240
   tgctgccctg acgatggcct ggagtgtgtg cccactgggc agcaccaagt ccggatgcag 300
   atcctcatga tccggtaccc gagcagtcag ctgggggaga tgtccctgga agaacacagc 360
   cagtgtgaat gcagacctaa aaaaaaggac agtgctgtga agccagacag ggctgccact 420
   ccccaccacc gtccccagcc ccgttctgtt ccgggctggg actctgcccc cggagcaccc 480
   tccccagctg acatcaccca tcccactcca gccccaggcc cctctgccca cgctgcaccc 540
   agcaccacca gcgccctgac ccccggacct gccgctgccg ctgccgacgc cgcagcttcc 600
   tccgttgcca agggcggggc ttag 624
<210> 11
   <211> 207
   <212> PRT
   <213> Artificial
<220>
   <223> VEGF-B isoform 1 amino acid sequence without KDEL
<400> 11
   Met Ser Pro Leu Leu Arg Arg Leu Leu Leu Ala Ala Leu Leu Gln Leu
   1               5                   10                  15
   Ala Pro Ala Gln Ala Pro Val Ser Gln Pro Asp Ala Pro Gly His Gln
               20                  25                  30
   Arg Lys Val Val Ser Trp Ile Asp Val Tyr Thr Arg Ala Thr Cys Gln
           35                  40                  45
   Pro Arg Glu Val Val Val Pro Leu Thr Val Glu Leu Met Gly Thr Val
       50                  55                  60
   Ala Lys Gln Leu Val Pro Ser Cys Val Thr Val Gln Arg Cys Gly Gly
   65                  70                  75                  80
   Cys Cys Pro Asp Asp Gly Leu Glu Cys Val Pro Thr Gly Gln His Gln
                   85                  90                  95
   Val Arg Met Gln Ile Leu Met Ile Arg Tyr Pro Ser Ser Gln Leu Gly
               100                 105                 110
   Glu Met Ser Leu Glu Glu His Ser Gln Cys Glu Cys Arg Pro Lys Lys
           115                 120                 125
   Lys Asp Ser Ala Val Lys Pro Asp Arg Ala Ala Thr Pro His His Arg
       130                 135                 140
   Pro Gln Pro Arg Ser Val Pro Gly Trp Asp Ser Ala Pro Gly Ala Pro
   145                 150                 155                 160
   Ser Pro Ala Asp Ile Thr His Pro Thr Pro Ala Pro Gly Pro Ser Ala
                   165                 170                 175
   His Ala Ala Pro Ser Thr Thr Ser Ala Leu Thr Pro Gly Pro Ala Ala
               180                 185                 190
   Ala Ala Ala Asp Ala Ala Ala Ser Ser Val Ala Lys Gly Gly Ala
           195                 200                 205
<210> 12
   <211> 211
   <212> PRT
   <213> Artificial
<220>
   <223> VEGF-B isoform 1 amino acid sequence with KDEL
<400> 12
   Met Ser Pro Leu Leu Arg Arg Leu Leu Leu Ala Ala Leu Leu Gln Leu
   1 5 10 15
   Ala Pro Ala Gln Ala Pro Val Ser Gln Pro Asp Ala Pro Gly His Gln
   20 25 30
   Arg Lys Val Val Ser Trp Ile Asp Val Tyr Thr Arg Ala Thr Cys Gln
   35 40 45
   Pro Arg Glu Val Val Val Pro Leu Thr Val Glu Leu Met Gly Thr Val
   50 55 60
   Ala Lys Gln Leu Val Pro Ser Cys Val Thr Val Gln Arg Cys Gly Gly
   65 70 75 80
   Cys Cys Pro Asp Asp Gly Leu Glu Cys Val Pro Thr Gly Gln His Gln
   85 90 95
   Val Arg Met Gln Ile Leu Met Ile Arg Tyr Pro Ser Ser Gln Leu Gly
   100 105 110
   Glu Met Ser Leu Glu Glu His Ser Gln Cys Glu Cys Arg Pro Lys Lys
   115 120 125
   Lys Asp Ser Ala Val Lys Pro Asp Arg Ala Ala Thr Pro His His Arg
   130 135 140
   Pro Gln Pro Arg Ser Val Pro Gly Trp Asp Ser Ala Pro Gly Ala Pro
   145 150 155 160
   Ser Pro Ala Asp Ile Thr His Pro Thr Pro Ala Pro Gly Pro Ser Ala
   165 170 175
   His Ala Ala Pro Ser Thr Thr Ser Ala Leu Thr Pro Gly Pro Ala Ala
   180 185 190
   Ala Ala Ala Asp Ala Ala Ala Ser Ser Val Ala Lys Gly Gly Ala Lys
   195 200 205
   Asp Glu Leu
   210
<210> 13
   <211> 567
   <212> DNA
   <213> Homo sapiens
<220>
   <223> VEGF-B isoform 2 nucleotide sequence for CDNA
<400> 13
   atgagccctc tgctccgccg cctgctgctc gccgcactcc tgcagctggc ccccgcccag      60
   gcccctgtct cccagcctga tgcccctggc caccagagga aagtggtgtc atggatagat     120
   gtgtatactc gcgctacctg ccagccccgg gaggtggtgg tgcccttgac tgtggagctc     180
   atgggcaccg tggccaaaca gctggtgccc agctgcgtga ctgtgcagcg ctgtggtggc     240
   tgctgccctg acgatggcct ggagtgtgtg cccactgggc agcaccaagt ccggatgcag     300
   atcctcatga tccggtaccc gagcagtcag ctgggggaga tgtccctgga agaacacagc     360
   cagtgtgaat gcagacctaa aaaaaaggac agtgctgtga agccagacag ccccaggccc     420
   ctctgcccac gctgcaccca gcaccaccag cgccctgacc cccggacctg ccgctgccgc     480
   tgccgacgcc gcagcttcct ccgttgccaa gggcggggct tagagctcaa cccagacacc     540
   tgcaggtgcc ggaagctgcg aaggtga                                         567
<210> 14
   <211> 188
   <212> PRT
   <213> Artificial
<220>
   <223> VEGF-B isoform 2 amino acid sequence without KDEL
<400> 14
   Met Ser Pro Leu Leu Arg Arg Leu Leu Leu Ala Ala Leu Leu Gln Leu
   1               5                   10                  15
   Ala Pro Ala Gln Ala Pro Val Ser Gln Pro Asp Ala Pro Gly His Gln
               20                  25                  30
   Arg Lys Val Val Ser Trp Ile Asp Val Tyr Thr Arg Ala Thr Cys Gln
           35                  40                  45
   Pro Arg Glu Val Val Val Pro Leu Thr Val Glu Leu Met Gly Thr Val
       50                  55                  60
   Ala Lys Gln Leu Val Pro Ser Cys Val Thr Val Gln Arg Cys Gly Gly
   65 70 75 80
   Cys Cys Pro Asp Asp Gly Leu Glu Cys Val Pro Thr Gly Gln His Gln
   85 90 95
   Val Arg Met Gln Ile Leu Met Ile Arg Tyr Pro Ser Ser Gln Leu Gly
   100 105 110
   Glu Met Ser Leu Glu Glu His Ser Gln Cys Glu Cys Arg Pro Lys Lys
   115 120 125
   Lys Asp Ser Ala Val Lys Pro Asp Ser Pro Arg Pro Leu Cys Pro Arg
   130 135 140
   Cys Thr Gln His His Gln Arg Pro Asp Pro Arg Thr Cys Arg Cys Arg
   145 150 155 160
   Cys Arg Arg Arg Ser Phe Leu Arg Cys Gln Gly Arg Gly Leu Glu Leu
   165 170 175
   Asn Pro Asp Thr Cys Arg Cys Arg Lys Leu Arg Arg
   180 185
<210> 15
   <211> 192
   <212> PRT
   <213> Artificial
<220>
   <223> VEGF-B isoform 2 amino acid sequence with KDEL
<400> 15
   Met Ser Pro Leu Leu Arg Arg Leu Leu Leu Ala Ala Leu Leu Gln Leu
   1 5 10 15
   Ala Pro Ala Gln Ala Pro Val Ser Gln Pro Asp Ala Pro Gly His Gln
   20 25 30
   Arg Lys Val Val Ser Trp Ile Asp Val Tyr Thr Arg Ala Thr Cys Gln
   35 40 45
   Pro Arg Glu Val Val Val Pro Leu Thr Val Glu Leu Met Gly Thr Val
   50 55 60
   Ala Lys Gln Leu Val Pro Ser Cys Val Thr Val Gln Arg Cys Gly Gly
   65 70 75 80
   Cys Cys Pro Asp Asp Gly Leu Glu Cys Val Pro Thr Gly Gln His Gln
   85 90 95
   Val Arg Met Gln Ile Leu Met Ile Arg Tyr Pro Ser Ser Gln Leu Gly
   100 105 110
   Glu Met Ser Leu Glu Glu His Ser Gln Cys Glu Cys Arg Pro Lys Lys
   115 120 125
   Lys Asp Ser Ala Val Lys Pro Asp Ser Pro Arg Pro Leu Cys Pro Arg
   130 135 140
   Cys Thr Gln His His Gln Arg Pro Asp Pro Arg Thr Cys Arg Cys Arg
   145 150 155 160
   Cys Arg Arg Arg Ser Phe Leu Arg Cys Gln Gly Arg Gly Leu Glu Leu
   165 170 175
   Asn Pro Asp Thr Cys Arg Cys Arg Lys Leu Arg Arg Lys Asp Glu Leu
   180 185 190
<210> 16
   <211> 5
   <212> PRT
   <213> Artificial
<220>
   <223> Lysozyme retention signal sequence (amino acid)
<400> 16
   Lys Phe Glu Arg Gln
   1 5
<210> 17
   <211> 15
   <212> DNA
   <213> Homo sapiens
<220>
   <223> Lysozyme retention signal sequence (nt)
<400> 17
   aarttygara grgar 15
<210> 18
   <211> 15
   <212> DNA
   <213> Homo Sapiens
<220>
   <223> Lysozyme retention signal sequence (nt)
<220>
   <221> misc_feature
   <222> (12)..(12)
   <223> n is a, c, g, or t
<400> 18
   aarttygarc gngar 15
<210> 19
   <211> 4
   <212> PRT
   <213> Artificial
<220>
   <223> Golgi retention signal sequence (nt)
<400> 19
   Tyr Gln Arg Leu
   1
<210> 20
   <211> 12
   <212> DNA
   <213> Homo Sapiens
<220>
   <223> Golgi retention signal sequence (nt)
<400> 20
   taycaragrt tr 12
<210> 21
   <211> 12
   <212> DNA
   <213> Homo Sapiens
<220>
   <223> Golgi retention signal sequence (nt)
<220>
   <221> misc_feature
   <222> (9)..(9)
   <223> n is a, c, g, or t
<400> 21
   taycarcgnt tr 12
<210> 22
   <211> 12
   <212> DNA
   <213> Homo sapiens
<220>
   <223> Golgi retention signal sequence (nt)
<220>
   <221> misc_feature
   <222> (12)..(12)
   <223> n is a, c, g, or t
<400> 22
   taycaragrc tn 12
<210> 23
   <211> 12
   <212> DNA
   <213> Homo sapiens
<220>
   <223> Golgi rentention signal sequence (nt)
<220>
   <221> misc_feature
   <222> (9)..(9)
   <223> n is a, c, g, or t
<220>
   <221> misc_feature
   <222> (12)..(12)
   <223> n is a, c, g, or t
<400> 23
   taycarcgnc tn 12
<210> 24
   <211> 4
   <212> PRT
   <213> Artificial
<220>
   <223> Yeast endoplasmic reticulum retention signal
<400> 24
   His Asp Glu Leu
   1

## Description

### FIELD OF THE INVENTION

The present invention relates to the fields of cell biology and oncology, and, more particularly, to VEGF binding members containing a cellular retention signal.

### BACKGROUND OF INVENTION

Tumor growth and metastasis is largely dependent on the degree of neovascularization in the tumor bed (Carmeliet, P et al., (2000) Nature 407, 249-57; Folkman, J (1995) Nat Med 1, 27-31; Hanahan, D. & Folkman, J. Cell (1996) 86, 353-64). Vascular endothelial growth factor (VEGF) is an important angiogenic factor that is frequently utilized by tumors and other tissues to switch on blood vessel growth (Dvorak., H.F. (2000) Semin Perinatol 24, 75-8; Ferrara, N. & Alitalo, K (1999) Nat Med 5, 1359-64; Yancopoulos, G.D. et al., (2000) Nature 407,242-8; Benjamin, L.E. & Keshet, E. (1997) Proc Natl Acad Sci USA 94, 8761-6). VEGF can also increase vascular permeability, which is important for tumor invasion and metastasis (Dvorak, H.F et al., (1999) Curr Top Microbiol Immunol 237, 97-132; Senger, D.R. et al., (1983) Science 219, 983-5). In addition to pathological angiogenesis, VEGF contributes to the development of the vascular system by stimulating vasculogenesis and angiogenesis during embryonic development (Carmeliet, P. et al., (1996) Nature 380, 435-9; Ferrara, N. et al., (1996) Nature 380,439-42).

VEGF members comprise structurally related members that include, but are not limited to, VEGF, the prototype of VEGF, placenta growth factor (PLGF), VEGF-B, VEGF-C, VEGF-D and VEGF-E (Eriksson, U. & Alitalo, K. (1999) Curr Top Microbiol Immunol 237, 41-57). The biological functions of the VEGF members are mediated by activation of at least three structurally homologous tyrosine kinase receptors, VEGFR-1/Flt-1, VEGFR-2/Flk-1/KDR and VEGFR-3/Flt-4 (Cao, Y. et al., (1998) Proc Natl Acad Sci USA 95, 14389-94). VEGF and PLGF also bind to a non-tyrosine kinase receptor neuropilin-1 (Migdal, M. et al., (1998). J Biol Chem 273, 22272-8; Soker, S et al., 1998) Cell 92, 735-45). According to their receptor binding patterns and angiogenic features, the VEGF members may be further divided into subgroups, such as, but not limited to,: 1) VEGF, which binds to VEGFR-1 and VEGFR-2, and induces vasculogenesis, angiogenesis and vascular permeability; 2) PLGF and VEGF-B, which selectively bind to VEGFR-1, and their physiological and pathological roles remain unknown; and 3) VEGF-C and VEGF-D, which interact with both VEGFR-2 and VEGFR-3, and induce both blood angiogenesis and lymphangiogenesis (Cao, Y. *et al.* supra; Makinen, T. et al., (2001) Nat Med 7, 199-205; Marconcini, L. et al., (1999) Proc Natl Acad Sci U S A 96, 9671-6; Skobe, M. et al, (2001) Nat Med 7, 192-8; Stacker, S.A. et al., (2001) Nat Med 7, 186-91). Accumulating evidence has suggested that VEGFR-2, in response to VEGF, mediates angiogenic signals for blood vessel growth and VEGFR-3 transduces signals for lymphatic vessel growth (Dvorak, H.F. supra; Ferrara, N. & Alitalo, K. supra; Ferrara, N. (1999) Curr Top Microbiol Immunol 237, 1-30).

Similar to the platelet growth factor (PDGF) members, VEGF members naturally exist as dimeric forms in order to interact with their specific receptors. In addition to homodimers, PLGF (SEQ ID NO: 2) and VEGF-B (SEQ ID NO: 11 or 14) can form heterodimers with VEGF when these factors are produced in the same cell (Cao, Y. et al., (1996) J Biol Chem 271, 3154-62; DiSalvo, J. et al., (1995) J Biol Chem 270, 7717-23. Distribution studies indicate that these factors are often expressed in overlapping tissues and cells. Thus, PLGF/VEGF or VEGF/VEGF-B heterodimers are naturally present in tissues when both factors are synthesized in the same population of cells (Cao, Y. *et al., supra;* Cao, Y. *et al.,* (1996) *supra*). These heterodimers demonstrate reduced angiogenic activity as compared to VEGF homodimers and may be used to inhibit angiogenesis as described in copending U.S. Application Serial No. 10/346,589 filed January 17,2003.

### SUMMARY OF THE INVENTION

An aspect of the invention provides an isolated nucleic acid comprising a cDNA sequence identical to a nucleic acid encoding a VEGF binding member with a cellular retention signal. The VEGF binding member is selected from PLGF (SEQ ID NO. 2), VEGF-B (SEQ ID NO. 11 or 14), VEGF receptor (VEGFR) 1 (VEGFR-1), VEGFR-2, neuropilin-1, and neuropilin-2. The cellular retention signal may comprise an endoplasmic reticulum retention signal, such as KDEL (SEQ ID NO: 7). Other suitable cellular retention signals include, but are not limited to, endoplasmic retention signal sequences, Golgi retention signal sequences (for example, but not limited to YQRL, (SEQ ID NO: 19)), endosome/lysosome retention sequences (for example, but not limited to KFERQ, (SEQ ID NO: 16)), mitochondria targeting sequences, nucleus targeting sequences, and/or peroxisome targeting sequences. Retention signals are well known to those knowledgeable in the art and the appropriate retention signal can readily be selected and incorporated into a protein coding sequence for translation of a protein with a retention signal. The cDNA sequence encoding a retention signal can be inserted just before the stop codon in the cDNA sequence encoding the protein to be retained.

Disclosed herein is a method for inhibiting the activity of VEGF (also referred to as VEGF-A) using an intracellular retention signal coupled to a VEGF binding protein. The agent may be used for treating a variety of diseases caused by VEGF-induced angiogenesis. One example of a method involves delivering a gene encoding a VEGF binding member selected from PLGF (SEQ ID NO: 1)., VEGF-B (SEQ ID NO: 10 or 13)., VEGF receptor (VEGFR) 1 (VEGFR-1), VEGFR-2, neuropilin-1, and neuropilin-2, to a cell which expresses VEGF, containing an intracellular retention signal, such that the binding member forms a heterodimer with VEGF when the two proteins are co-expressed in the cell. As demonstrated by the studies described herein, heterodimers of VEGF/PLGF and VEGF/VEGF-B have reduced angiogenic activity compared to VEGF/VEGF homodimers and, thus, inhibit the angiogenic activity of VEGF.

In a particular embodiment of the invention, the gene encoding the VEGF binding member with an intracellular retention signal is contained within a vector suitable for gene delivery. Such vectors include, for example, adenoviral vectors, retroviral vectors, lentiviral vectors, vaccinia viral vectors, adeno-associated viral vectors, RNA vectors, liposomes, cationic lipids, transposons and the like. In a preferred embodiment, the gene is contained within a retroviral vector or a lentiviral vector. The gene can be also be delivered or co-administered with another anti-angiogenic agent or anti-cancer agent.

Disclosed herein is a method that can be used *in vitro* or *ex vivo* to inhibit angiogenesis, diabetic retinopathy and/or tumor growth. The method also can be used *in vivo* to treat a variety of diseases involving VEGF-induced angiogenesis in subjects including animals and humans. Such diseases include, for example, a variety of cancers, diabetic retinopathy and autoimmune diseases, such as rheumatoid arthritis.

Further uses for the present invention include, but are not limited to, the development of a growth factor antagonist(s) by sequestration of the growth factor within a cell by a growth factor binding member(s) (proteins, glycans or lipid, for example). An intracellular retention signal will function to affect the intracellular routing of the protein. The ER retention signal KDEL (SEQ ID NO: 7), a mammalian ER retention signal sequence, is an example of the retention signal that may be used in the present invention. Other suitable endoplasmic retention signals may include, but are not limited to, functionally equivalent amino acid sequences such as those derivable from a bacterial toxin, such as ETA, or from a yeast, such as HDEL (SEQ ID NO: 24).

A further embodiment of the invention is an isolated nucleic acid comprising a nucleotide sequence substantially identical to a nucleic acid encoding a VEGF binding member with an intracellular retention signal. The isolated nucleic encoding the VEGF binding member includes, but is not limited to, VEGF-B (SEQ ID NO: 10 or 13), or PLGF-1 (SEQ ID NO: 1). In one embodiment the cellular retention signal can be an endoplasmic reticular retention signal comprising KDEL (SEQ ID NO: 7).

Another aspect of the present invention is a recombinant plasmid encoding nucleic acid sequences for expressing a VEGF binding member with an intracellular retention signal, wherein the VEGF binding member with an intracellular retention signal forms a heterodimer with an intracellular VEGF. A further aspect of the invention is a recombinant viral vector encoding a nucleotide sequence that encodes a VEGF binding member with an intracellular retention signal, which forms a heterodimer with an intracellular VEGF and wherein the nucleotide sequence is a cDNA sequence. The VEGF binding member nucleotide sequence in the recombinant viral vector can be VEGF-B (SEQ ID NO: 10 or 13) or PLGF-1 (SEQ ID NO: 1). The endoplasmic reticular retention signal can be KDEL (SEQ ID NO: 7). The recombinant viral vector can be an adenoviral vector, an adeno- associated viral vector, a lentiviral vector, a retroviral vector, a herpes virus vector or the like.

An embodiment of the invention is a pharmaceutical composition comprising a viral vector containing a gene encoding a VEGF binding member with an intracellular retention signal and a pharmaceutically acceptable carrier.

Also disclosed herein is a method of inhibiting secretion of VEGF by administering to a patient a retroviral vector in an amount sufficient to transduce VEGF-secreting cells in the patient, wherein the retroviral vector contains a nucleotide sequence encoding a VEGF binding member with a cellular retention signal and wherein the VEGF binding member with a cellular retention signal is expressed in an amount effective to bind to the VEGF to form a heterodimers, and for inhibition of VEGF secretion from a cell. Embodiments of the invention include aspects where the patient has a disease selected from the group consisting of cancers, inflammatory arthritis (such as rheumatoid arthritis), diabetic retinopathy, as well as other neovascular diseases of the eye (for example, corneal neovascularization, neovascular glaucoma, retrolental fibroblasia and macular degeneration), arteriovenous malformations, conditions of excessive bleeding (menorrhagia), Osler-Webber Syndrome, myocardial angiogenesis, plaque neovascularization, telangiectasia, hemophiliac joints, angiofibroma, wound granulation, and diseases of excessive or abnormal stimulation of endothelial cells. The VEGF binding member with a cellular retention signal can be VEGF-B (SEQ ID NO: 12 or 15) or PLGF-1 (SEQ ID NO: 3). The cellular retention signal can be KDEL (SEQ ID NO: 7).

Another embodiment is a pharmaceutical composition with at least one retroviral vector containing a nucleotide sequence encoding a VEGF binding member with a cellular retention signal. The VEGF binding member with a cellular retention signal can VEGF-B (SEQ ID NO: 12 or 15) or PLGF-1(SEQ ID NO: 3). The cellular retention signal can be KDEL (SEQ ID NO: 7).

### BRIEF DESCRIPTION OF FIGURES

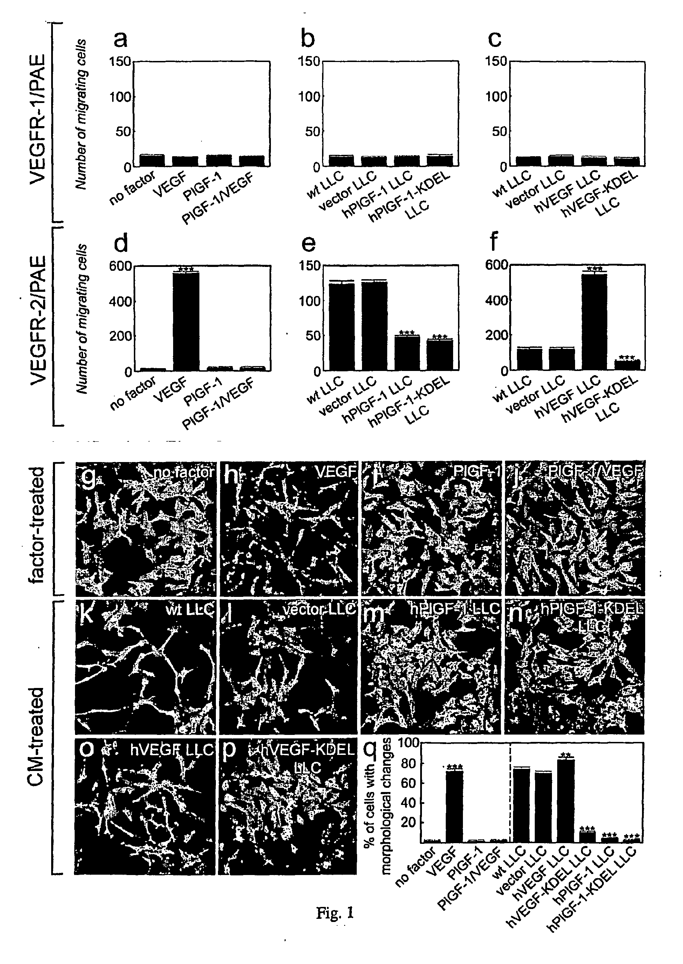

Fig. 1. Chemotactic activity and cell shape changes/actin reorganization produced by various tumor cells lines. Purified PLGF-1 (SEQ ID NO: 2), VEGF, PLGF-1/VEGF heterodimers (a, d, h, i, and j), or conditioned media from various transduced and non-transdcued LLC tumor cell lines (b, c, e, f, and k-p) were assayed for their chemotactic (a-f) and cell morphological (g-p) effects on VEGFR-1/PAE (a-c) and VEGFR-2/PAE (d-q) endothelial cells. Migrating cells were counted per optic field (32x) and data represent mean % (±SEM) of a quadruplicate of each sample (a-f). For Morphological analysis, growth factors or conditioned media were incubated with VEGFR-2/PAE cells and actin reorganization was examined by TRITC-phalloidin staining (g-p). Spindle-like cells were counted from 5 random optical fields (20x) of each sample, and are presented as average percentages of total cell numbers (±SEM) (q). **P*<0.05; ** *P*<0.01; and *P*<0.001.

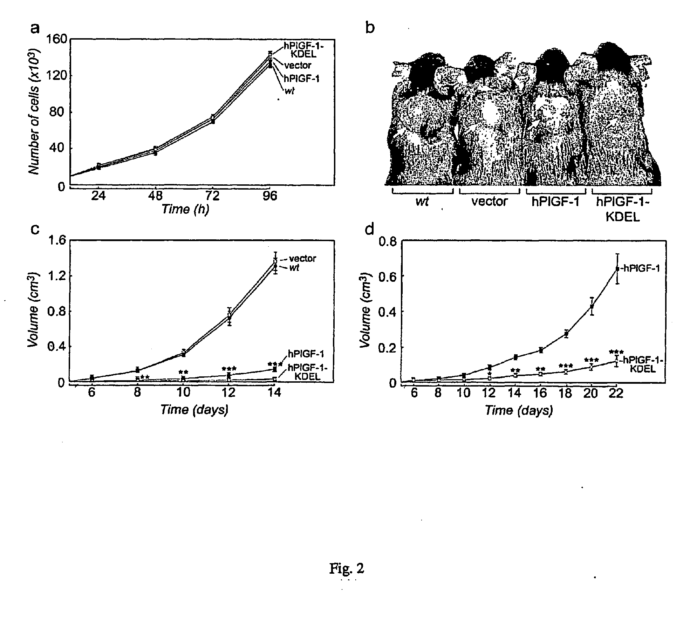

Fig. 2. Suppression of tumor growth by PLGF-1-KDEL (SEQ ID NO.: 3) a, growth rates of *wt* LLC, vector-LLC, hPLGF-1-LLC, and hPLGF-1-KDEL-LLC tumor cells at indicated time points. Approximately, 1 x 10⁶ tumor cells of each cell line were subcutaneously injected into 6-week-old C57B 1/6 female mice. Tumor sizes were measured every other day starting at day 6 (c and d). At day 14 after tumor cell implantation, typical tumor appearances were photographed (b) and tumor volumes were measured according the standard formula: *length x width² x 0.52.* Yellow arrows indicate the implanted tumors. Data are presented as mean % (±SEM) of six mice in each group (c and d). For comparison of hPLGF-1-KDEL-LLC tumors with hPLGF-1 and control tumors (b-d); **P*<0.05; ***P*<0.01; and *** *P*<0.001.

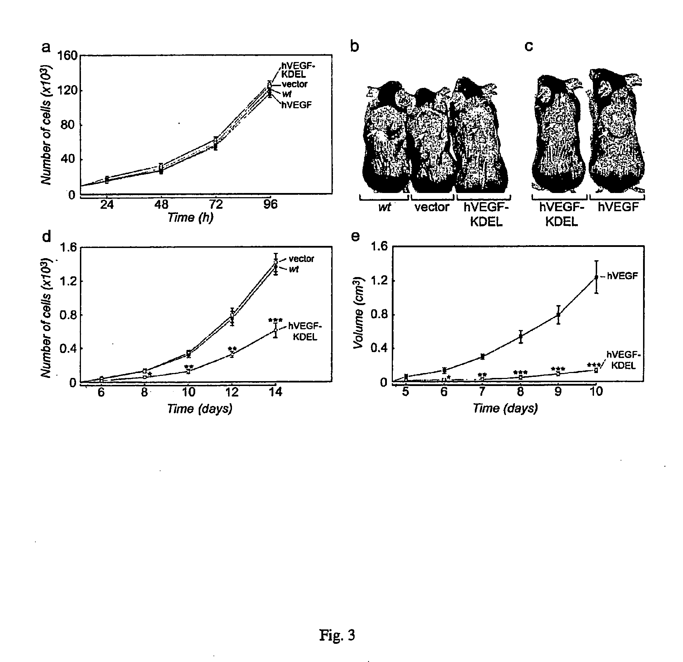

Fig. 3. Suppression of tumor growth by hVEGF-KDEL a, growth rates of *wt* LLC, vector-LLC, hVEGF-LLC, and hVEGF-KDEL-LLC tumor cells at indicated time points. Approximately, 1 x 10⁶ tumor cells of each cell line were subcutaneously injected into 6-week-old C57B 1/6 female mice. Tumor sizes were measured every other day starting at day 5 (d). At day 14 (b) and day 10 (c) after tumor implantation, typical tumor appearances were photographed and tumor volumes were measured according the standard formula: *length x width² x 0.52.* Yellow arrows point to the implanted tumors. Data are presented as mean % (±SEM) of six mice in each group (d and e). For comparison of hVEGF-KDEL-LLC tumors with hVEGF (c and e) and control tumors (b and d); **p*<0.05; ** *p*<0.01; and *** *p*<0.001.
Fig. 4. Immunohistochemical detection of tumor vessel *wt* LLC, *vector*-LLC, hVEGF-LLC, hVEGF-KDEL-LLC, hPLGF- 1 -LLC, and hPLGF- 1 -KDEL-LLC-tumors were grown to a similar size and histological sections were stained with an anti-CD31 antibody using a conventional immunohistochemical method (a-f) or a wholemount/confocal method (h-m). Microvessel density was counted under a light microscope in at least 6 random fields (20x) and are presented as mean values (±SEM) (g).** *p*<0.01; and *** *p*<0.001.

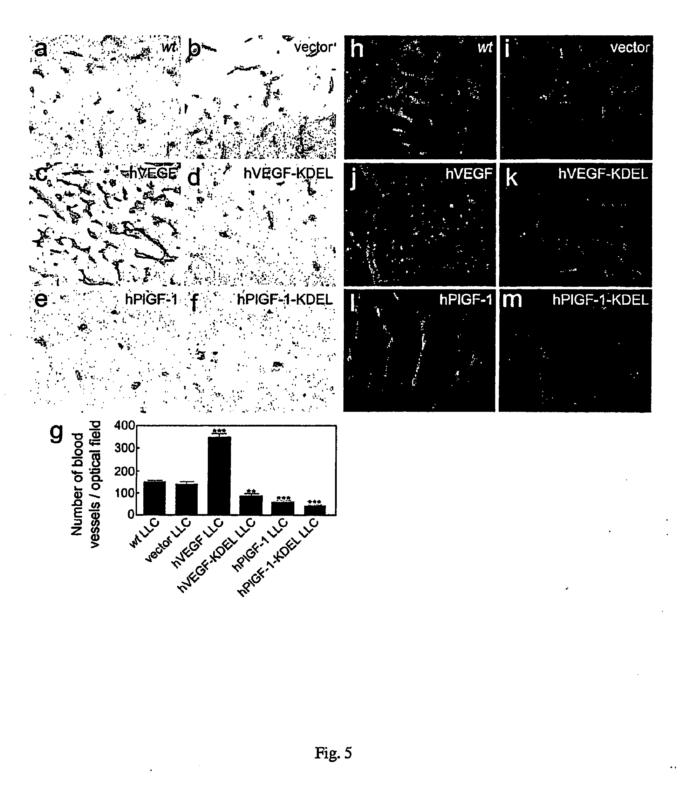

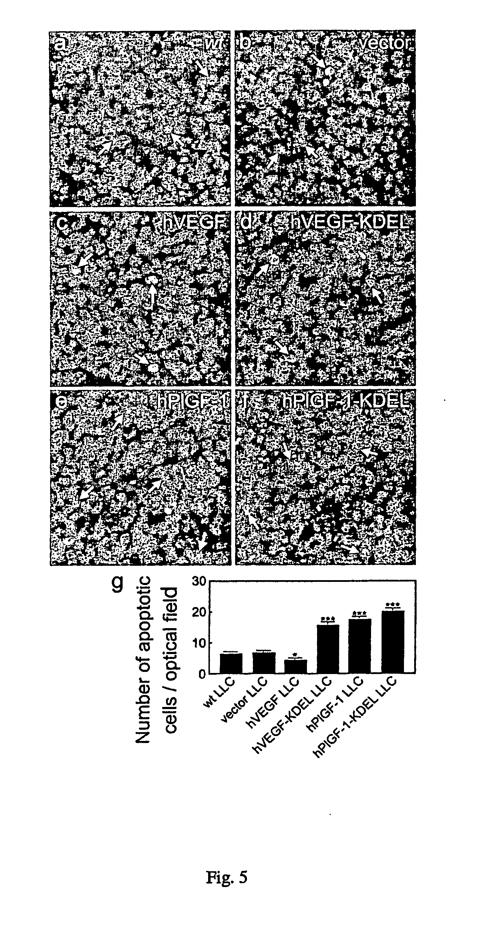

Fig. 5. Tumor cell apoptosis Histological sections of *wt* LLC, vector-LLC, hVEGF-LLC, hVEGF-KDEL-LLC, hPLGF-1-LLC, and hPLGF1-KDEL-LLC-tumors were stained with a FITC-labelled TUNEL kit, followed by counter-staining with Hoescht dye (blue). Arrows point to apoptotic green cells (a-f). The number of apoptotic cells was quantified in 10 randomly chosen optical fields (40x), and mean values are presented (±SEM) (g). **P*<0.05; ***P*<0.01; and ****P*<0.001.

### DETAILED DESCRIPTION OF THE INVENTION

Before the present compositions and methods are described, it is to be understood that this invention is not limited to the particular molecules, compositions, methodologies or protocols described, as these may vary. It is also to be understood that the terminology used in the description is for the purpose of describing the particular versions or embodiments only, and is not intended to limit the scope of the present invention which will be limited only by the appended claims.

The terms used herein have meanings recognized and known to those of skill in the art, however, for convenience and completeness, particular terms and their meanings are set forth below.

It must also be noted that as used herein and in the appended claims, the singular forms "a", "an", and "the" include plural reference unless the context clearly dictates otherwise. Thus, for example, reference to a "spheroid" is a reference to one or more spheroid and equivalents thereof known to those skilled in the art, and so forth. Unless defined otherwise, all technical and scientific terms used herein have the same meanings as commonly understood by one of ordinary skill in the art. Although any methods and materials similar or equivalent to those described herein can be used in the practice or testing of embodiments of the present invention, the preferred methods, devices, and materials are now described. Nothing herein is to be construed as an admission that the invention is not entitled to antedate such disclosure by virtue of prior invention.

The terms "patient" and "subject" mean all animals including humans. Examples of patients or subjects include humans, cows, dogs, cats, goats, sheep, and pigs.

The term "angiogenesis" refers to the generation of new blood supply, e.g., blood capillaries, vessels, and veins, from existing blood vessel tissue (e.g., vasculature). The process of angiogenesis can involve a number of tissue cell types including, for example, endothelial cells which form a single cell layer lining of all blood vessels and are involved with regulating exchanges between the bloodstream and the surrounding tissues. New blood vessels (angiogenesis) can develop from the walls of existing small vessels by the outgrowth of endothelial cells. Angiogenesis is also involved in tumor growth as it provides tumors with blood supply necessary for tumor cell survival and proliferation (growth).

The term "cancer" refers to any malignant growth or tumor caused by abnormal and uncontrolled cell division; it may spread to other parts of the body through the lymphatic system or the blood stream. Cancer include both solid tumors and blood-borne tumors. Solid tumors include, or example, but not limited to Kaposi's sarcoma, hemangiomas, solid tumors, blood-borne tumors, breast cancer, lung cancer, ovarian cancer, testicular cancer, colon cancer, rhabdomyosarcoma, retinoblastoma, Ewing's sarcoma, neuroblastoma, and osteosarcoma. Angiogenesis is also associated with blood-borne tumors, such as leukemias, any of various acute or chronic neoplastic diseases of the bone marrow in which unrestrained proliferation of white blood cells occurs, usually accompanied by anemia, impaired blood clotting, and enlargement of the lymph nodes, liver and spleen. It is believed to that angiogenesis plays a role in the abnormalities in the bone marrow that give rise to leukemia-like tumors.

The term "inhibiting angiogenesis" as used herein refers to complete or partial inhibition of angiogensis.

The term "gene" as used herein refers to DNA (cDNA) or RNA encoding a protein of interest, such as PLGF (SEQ ID NO: 3) or VEGF-B (SEQ ID NO: 12 or 15) containing sequences coding for KDEL (SEQ ID NO: 7) at the 3' end of the coding sequence before the stop codon. The DNA gene is a complementary DNA (cDNA) sequence, thus lacking intron sequences and containing those sequences that encode the protein of interest (i.e., VEGF binding member with an intracellular retention signal). Genes encoding VEGF binding members used in the present invention are typically contained within an expression vector along with genetic elements necessary for expression of the gene by a cell. Such elements are well known in the art and include, for example, suitable promoters and enhancers.

The term "VEGF binding member" refers to a protein or peptide other than VEGF which binds to VEGF (also referred to as "VEGF-A") and inhibits VEGF activity (e.g., VEGF-induced angiogenesis) as measured, for example, by the numerous VEGF activity assays described herein. VEGF binding members include, for example, PLGF, VEGF-B, and other proteins which naturally bind to VEGF and, optionally, also to VEGFR-1 (as does VEGF).

The terms "PLGF" and "VEGF-B" refer to PLGF and VEGF-B growth factors as well as functionally equivalent analogs that bind to (form heterodimers with) VEGF and reduce the activity of VEGF. Functionally equivalent analogs include, for example, functionally equivalent peptides or homologues derived from PLGF and/or VEGF-B that retain the ability to bind to VEGF and to reduce its activity compared to cells in which the PLGF, VEGF-B or analog thereof has not been delivered.

By "functionally equivalent" refers to a composition that has anti-angiogenic activity, and behaves similarly the VEGF binding members with a cellular retention signal, as determined in standard assays. "Standard assays" include, but are not limited to, those protocols used in the molecular biological arts to assess anti-angiogenic activity, cell shape assay and actin staining, cell cycle arrest, detection of apoptosis, chemotaxis assay, and endothelial cell migration. Such assays are provided in the Examples contained herein.

The term "expressed at sufficient levels" in reference to VEGF binding members (for example, PLGF and VEGF-B) refers to levels necessary to partially or fully inhibit VEGF activity (e.g., VEGF-induced angiogenesis). The VEGF binding member is preferably expressed at levels which are equal (e.g., a 1:1 ratio) or, more preferably, which are greater than the level of endogenous VEGF expressed within the cell, so that VEGF/VEGF binding member containing KDEL (SEQ ID NO: 7) heterodimers are formed within the cell at greater levels than VEGF/VEGF homodimers. For example, the VEGF binding member can be expressed at a ratio of 1:2. 1:3, 1:4, 1:5, 1:6, 1:7 or higher with respect to the level of VEGF expressed in the celL This level of expression reduces the overall activity of VEGF that would occur in the absence of expressing the VEGF binding member and is referred to as "over-expression" of the VEGF binding member. Moreover, in some cases (depending on the cells being treated), the VEGF binding member may already be expressed naturally (endogenously) within the cell, such that delivery of the gene encoding the VEGF binding member to the cell increases the overall level of VEGF binding member expression to a level which reduces or blocks VEGF activity.

A "therapeutically effective amount" in reference to pharmaceutical compositions is an amount sufficient to decrease or prevent the symptoms associated with a medical condition or infirmity, to normalize body functions in disease or disorders that result in impairment of specific bodily functions, or to provide improvement in one or more of the clinically measured parameters of the disease. As related to the present application, a therapeutically effective amount is an amount sufficient to decrease or inhibit secretion or expression of VEGF or inhibit or decrease angiogenesis.

Assays for measuring or quantifying protein levels (e.g., standard ELISA assays) of VEGF binding member compared to VEGF, and for measuring VEGF activity are well known in the art and include, for example, those described in the studies provided herein.

The term "retroviral vector" refers to a vector containing structural and functional genetic elements that are primarily derived from a retrovirus, such as type c retroviruses. Suitable retroviral vectors include, for example, Moloney murine sarcoma virus (MoMSV), Harvey murine sarcoma virus (HaMuSV), murine mammary tumor virus (MuMTV), gibbon ape leukemia virus (GaLV), feline leukemia virus (FLY), spumavirus, Friend, Murine Stem Cell Virus (MSCV) and Rous Sarcoma Virus (RSV)). "Retroviral vectors" used in the invention can also include vectors derived from human T cell leukemia viruses, HTLV-1 and HTLV-2, and the lentiviral family of retroviruses, such as human Immunodeficiency viruses, HIV-1, HIV-2, simian immnodeficiency virus (S1Y), feline immonodeficiency virus (F1Y), equine immnodeficiency virus (E1Y), and other classes of retroviruses.

"Retroviruses" are RNA viruses that utilize reverse transcriptase during their replication cycle. The retroviral genomic RNA is converted into double-stranded DNA by reverse transcriptase. This double-stranded DNA form of the virus is capable of being integrated into the chromosome of the infected cell; once integrated, it is referred to as a "provirus." The provirus serves as a template for RNA polymerase II and for the expression of mRNA molecules which encode the structural proteins and enzymes needed to produce new viral particles. At each end of the provirus are structures called "long terminal repeats" or "LTRs." The LTR contains numerous regulatory signals including transcriptional control elements, polyadenylation signals and sequences needed for replication and integration of the viral genome. The viral LTR is divided into three regions called U3, R and U5. The U3 region contains the enhancer and promoter elements. The U5 region is the sequence between the primer binding site and the R region and contains the polyadenylation sequence. The R (repeat) region is flanked by the U3 and U5 regions. The LTR composed of U3, R and U5 regions, appears at both the 5' and 3' ends of the viral genome.

The term "lentivirus" refers to a group (or genus) of retroviruses that give rise to slowly developing disease. Viruses included within this group include HIV (human immunodeficiency virus; including HIV type 1, and HIV type 2), the etiologic agent of the human acquired immunodeficiency syndrome (AIDS); visna-maedi, which causes encephalitis (visna) or pneumonia (maedi) in sheep, the caprine arthritis-encephalitis virus, which causes immune deficiency, arthritis, and encephalopathy in goats; equine infectious anemia virus, which causes autoimmune hemolytic anemia, and encephalopathy in horses; feline immunodeficiency virus (FIV), which causes immune deficiency in cats; bovine immune deficiency virus (BIV), which causes lymphadenopathy, lymphocytosis, and possibly central nervous system infection in cattle; and simian immunodeficiency virus (SIV), which cause immune deficiency and encephalopathy in sub-human primates. Diseases caused by these viruses are characterized by a long incubation period and protracted course. Usually, the viruses latently infect monocytes and macrophages, from which they spread to other cells. HIV, FIV, and SIV also readily infect T lymphocytes (i.e., T-cells).

The term "vector" refers to a nucleic acid molecule capable of transporting (e.g., into a cell) another nucleic acid to which it has been linked. The term "expression vector" includes any vector, (e.g., a plasmid, cosmid or phage chromosome) containing a gene construct in a form suitable for expression by a cell (e.g., linked to a promoter). In the present specification, "plasmid" and "vector" are used interchangeably, as a plasmid is a commonly used form of vectors. Moreover, the invention is intended to include other vectors which serve equivalent functions.

The term "gene delivery" or "transfection" refers to the introduction of exogenous DNA or RNA into eukaryotic cells. Gene delivery in the present invention can be accomplished *in vitro, in vivo* and *ex vivo* using any of a variety of means well known in the art. For example, *in vitro* and *ex vivo* gene delivery can be accomplished using techniques such as calcium phosphate-DNA co-precipitation, DEAE-dextranmediated transfection, polybrene-mediated transfection, electroporation, microinjection, liposome fusion, lipofection, protoplast fusion, biolistics and viral transduction. *In vivo* gene delivery can be achieved using a variety of art-recognized techniques including, most commonly, injection (e.g., intravenous, intramuscular etc.).

An aspect of the present invention is based on the discovery that PLGF with an ER retention signal, such as KDEL (SEQ ID NO: 7), at the C-terminus can inhibit the function of VEGF (i.e., "VEGF-A) when both factors are produced in the same population of cells, and that the underlying mechanism is can be due to the formation of VEGF heterodimers. Heterodimer formation between VEGF and a genetically engineered PLGF (SEQ ID NO: 3) containing an ER retention signal at the C-terminus will inhibit the angiogenic activity of VEGF.

The VEGF binding member with a cellular retention signal may be administered by delivery of a gene encoding the VEGF binding member with a cellular retention signal within a viral vector, preferably within a retroviral or lentiviral vector. A particular lentiviral vector which can be used in the present invention includes the self-inactivating lentiviral vector described in U.S. Provisional Patent Application Serial No. 60/288,042.

In further embodiments of the invention, vectors (e.g., retroviral and lentiviral vectors) for gene delivery also can be incorporated into virions using packaging cell lines prior to contact with a cell, as is well known in the art The phrase "packaging cell line" refers to a cell line (typically a mammalian cell line) which contains the necessary coding sequences to produce viral particles which lack the ability to package DNA or RNA and produce replication-competent helper-virus. When the packaging function is provided within the cell line (e.g., in *trans* by way of a plasmid vector), the packaging cell line produces recombinant virus, thereby becoming a "producer cell line." Any suitable packaging cell line can be used in the present invention depending on the nature of the vector.

In addition, the gene encoding the VEGF binding members, such as PLGF-KDEL (SEQ ID NO: 3) and VEGF-B-KDEL (SEQ ID NO: 12 or 15), can be delivered either alone or in combination with one or more other angiogenesis-inhibiting ("anti-angiogenic") factor(s), including, for example, but not limited to, endostatin or angiostatin (see e.g., U.S Patent No.: 6,174,861 and 6,024,688), or one or more anti-cancer agents, such as chemotherapeutic agents or radiation. Moreover, multiple genes encoding different VEGF binding members with intracellular retention signals can be concurrently delivered to enhance VEGF inhibition.

Intracellular retention signals include, but are not limited, to endoplasmic retention signal sequences, Golgi retention signal sequences, endosome/lysosome retention sequences, mitochondria targeting sequences, nucleus targeting sequences, and/or peroxisome targeting sequences. These retention signals can be located in any position of the VEGF-binding protein sequences so long as at least a portion of the functional activity of the protein is retained. The portion of functional activity retained, in one embodiment, is at least 50% that of the protein native protein. In a further embodiment the portion of the functional activity retained is at least 75% that of the protein native protein. In another embodiment the portion of the functional activity retained is at least 90% that of the protein native protein.

Accordingly, disclosed herein are methods for treating diseases caused by VEGF activity and VEGF-induced angiogenesis (e.g., cancer, diabetic retinopathy, and rheumatoid arthritis) using gene therapy and a variety of gene delivery systems, such as retroviral and lentiviral gene delivery systems. Such systems provide a sustained, high-level expression of transferred therapeutic genes *in vivo,* and are highly efficient at infecting and integrating in a non-toxic manner into the genome of a wide variety of cell types.

A wide variety of diseases are the result of undesirable angiogenesis. Thus, many diseases and undesirable conditions could be prevented or alleviated if it were possible to stop the growth and extension of capillary blood vessels under some conditions, at certain times, or in particular tissues. Angiogenesis-dependent diseases that can be treated by the invention disclosed herein are those conditions/diseases which require or induce vascular growth. For example, cancers, inflammatory arthritis (such as rheumatoid arthritis), diabetic retinopathy, as well as other neovascular diseases of the eye (or example, corneal neovascularization, neovascular glaucoma, retrolental fibroblasia and macular degeneration), arteriovenous malformations, conditions of excessive bleeding (menorrhagia), Osler-Webber Syndrome, myocardial angiogenesis, plaque neovascularization, telangiectasia, hemophiliac joints, angiofibroma, and wound granulation. The anti-angiogenic compositions provided herein are useful in the treatment of diseases of excessive or abnormal stimulation of endothelial cells. These diseases include, but are not limited to, intestinal adhesions, Crohn's disease, atherosclerosis, scleroderma, and hypertrophic scars (i.e., keloids).

Although the invention has been described with reference to its preferred embodiments, other embodiments can achieve the same results. Those skilled in the art will recognize or be able to ascertain using no more than routine experimentation, numerous equivalents to the specific embodiments described herein. Such equivalents are considered to be within the scope of this invention and are encompassed by the following paragraphs.

### Gene Therapy

Gene therapy refers to therapy performed by the administration of a nucleic acid to a subject. In gene therapy disclosed herein, a nucleic acid sequence encoding a VEGF binding member with an intracellular retention signal is delivered to a cell and mediates a therapeutic effect by inhibiting secretion of VEGF. In a gene therapy protocol, a vector containing an expression cassette which encodes for a VEGF binding member with an intracellular retention signal, such as VEGF-KDEL, PLGF-1-KDEL (SEQ ID NO: 3), can be directly administered to a cell, such as a tumor cell, for the inhibition of VEGF secretion. Recently, there has been a lot of activity in synthesizing retroviral vectors with chimeric coat proteins. The chimeric proteins typically comprise two domains, one of which is embedded in the viral coat and is of retroviral origin. The second domain is heterologous to the virus and is a member of a binding pair. For example, the second domain consists of a single chain Fv fragment which binds to a tumor cell surface marker or it is the ligand to which an antibody expressed on the cell surface binds. Other binding pairs, not necessarily monoclonal antibodies and their ligands will be apparent to those of skill. These ligands will target the gene therapy to the appropriate cells.

Any of the methods for gene therapy available in the art can be used according to the present invention. Exemplary methods are described below.

For general reviews of the methods of gene therapy, see Goldspiel et al., 1993, Clinical Pharmacy 12:488-505; Wu and Wu, 1991, Biotherapy 3:87-95; Tolstoshev, 1993, Ann. Rev. Pharmacol. Toxicol. 32:573-596; Mulligan, 1993, Science 260:926-932; and Morgan and Anderson, 1993, Ann. Rev. Biochem. 62:191-217; May, 1993, TIBTECH 11(5): 155-215). Methods commonly known in the art of recombinant DNA technology which can be used are described in Ausubel et al. (eds.), 1993, Current Protocols in Molecular Biology, John Wiley & Sons, NY; and Kriegler, 1990, Gene Transfer and Expression, A Laboratory Manual, Stockton Press, NY.

In one embodiment, the pharmaceutical composition (*infra*) comprises a VEGF binding member with an intracellular retention signal nucleic acid as part of an expression vector that expresses a VEGF binding member with an intracellular retention signal protein. In particular, such a nucleic acid has a promoter operably linked to the coding region, the promoter being inducible or constitutive, and, optionally, tissue-specific. In another particular embodiment, a nucleic acid molecule is used in which the aforementioned binding member coding sequences and any other desired sequences are flanked by regions that promote homologous recombination at a desired site in the genome, thus providing for intrachromosomal expression of the nucleic acid

Delivery of the nucleic acid into a patient may be either direct, in which case the patient is directly exposed to the nucleic acid or nucleic acid-carrying vector, or indirect, in which case, cells are first transformed with the nucleic acid in vitro, then transplanted into the patient. These two approaches are known, respectively, as in vivo or ex vivo gene therapy.

In a specific embodiment, the nucleic acid is for being directly administered in vivo, where it is expressed to produce the encoded product. This can be accomplished by any of numerous methods known in the art, e.g., by constructing it as part of an appropriate nucleic acid expression vector and administering it so that it becomes intracellular, e.g., by infection using a defective or attenuated retroviral or other viral vector (see U.S. Pat. No. 4,980,286), or by direct injection of naked DNA, or by use of microparticle bombardment (e.g., a gene gun; Biolistic, Dupont), or coating with lipids or cell-surface receptors or transfecting agents, encapsulation in liposomes, microparticles, or microcapsules, or by administering it in linkage to a peptide which is known to enter the nucleus, by administering it in linkage to a ligand subject to receptor-mediated endocytosis (see e.g., Wu and Wu, 1987, J. Biol. Chem. 262:4429-4432) (which can be used to target cell types specifically expressing the receptors), etc.

In another embodiment, a nucleic acid-ligand complex can be formed in which the ligand is a fusogenic viral peptide to disrupt endosomes, allowing the nucleic acid to avoid lysosomal degradation. In yet another embodiment, the nucleic acid can be targeted in vivo for cell specific uptake and expression by targeting a specific receptor ((*supra* and see, e.g., PCT Publications WO 92/06180 dated Apr. 16, 1992 (Wu et al.); WO 92/22635 dated Dec. 23, 1992 (Wilson et al.); WO92/20316 dated Nov. 26, 1992 (Findeis et al.); WO93/14188 dated Jul. 22,1993 (Clarke et al.), WO 93/20221 dated Oct. 14, 1993 (Young)). Alternatively, the nucleic acid can be introduced intracellularly and incorporated within host cell DNA for expression, by homologous recombination (Koller and Smithies, 1989, Proc. Natl. Acad. Sci. USA 86:8932-8935; Zijlstra et al., 1989, Nature 342:435-438).

### Retroviral vectors encoring a VEGF binding member with an intracellular retention signal

In a specific embodiment, a viral vector that contains nucleic acid encoding the VEGF binding member with an intracellular retention signal is used. For example, a retroviral vector can be used (see Miller et al., 1993, Meth. Enzymol. 217:581-599). These retroviral vectors have been modified to delete retroviral sequences that are not necessary for packaging of the viral genome and integration into host cell DNA. A nucleic acid encoding the VEGF binding member with an intracellular retention signal to be used in gene therapy is cloned into the vector, which facilitates delivery of the gene into a patient. References illustrating the use of retroviral vectors in gene therapy are: Clowes et al., 1994, J. Clin. Invest. 93:644-651; Kiem et al., 1994, Blood 83:1467-1473; Salmons and Gunzberg, 1993, Human Gene Therapy 4:129-141; and Grossman and Wilson, 1993, Curr. Opin. in Genetics and Devel. 3:110-114.

Retroviral vectors are useful as agents to mediate retroviral-mediated gene transfer into eukaryotic cells. Retroviral vectors are generally constructed such that the majority of sequences coding for the structural genes of the virus are deleted and replaced by the gene(s) of interest. Most often, the structural genes (i.e., gag, pol, and env), are removed from the retroviral backbone using genetic engineering techniques known in the art.

The removal of the gag, pol and env genes results in a vector backbone, comprised of a 5' LTR, a packaging signal, one or more cloning sites, into which the heterologous gene or genes of interest can be introduced, and a 3' LTR. An example of a vector backbone that can be used is the G1 vector backbone, which is disclosed in McLachlin, et al., Virology, 195:1-5 (1993) and in PCT patent application no. WO 91/10728 for "Novel Retroviral Vectors," published on Jul. 25, 1991.

The heterologous gene or genes are incorporated into the proviral backbone by standard techniques to form the retroviral vector. Techniques for the preparation of retroviral vectors are disclosed in PCT application WO 91/10728 as well as the following articles: Armentano, et al., J. Virol., 61:1647-1650 (1987), Bender, et al., J. Virol., 61:1639-1646 (1987), and Miller, et al., Biotechniques, 7:980-990 (1989). The most straightforward constructions are ones in which the structural genes of the retrovirus are replaced by a single gene which then is transcribed under the control of the viral regulatory sequences within the long terminal repeat (LTR). Retroviral vectors have also been constructed which can introduce more than one gene into target cells. Usually, in such vectors one gene is under the regulatory control of the viral LTR, while the second gene is expressed either off a spliced message or is under the regulation of its own, internal promoter. Suitable promoters include the SV40 promoter, the human cytomegalovirus (CMV) promoter, the beta-actin promoter, the alpha fetoprotein promoter, and any promoter naturally associated with any heterologous gene of interest.

The retroviral vectors may be in the form of a plasmid, a segment of viral RNA, or a segment of proviral DNA. The retroviral vector is introduced into a packaging cell to form a producer cell. Packaging cells provide the gag, pol, and env genes in trans, which permits the packaging of the retroviral vector into a recombinant retrovirus that is infectious but replication defective. The vectors are transferred into the packaging cells by standard gene transfer techniques, which include transfection, transduction, calcium phosphate precipitation, electroporation, and liposome-mediated DNA transfer. Examples of packaging cells that may be used include, but are not limited to, the PE501, PA317, Psi-2, Psi-AM, PA12, T19-14X, VT-19-17-H2, Psi-CRE, Psi-CRIP, GP+E-86, GP+envAM12, and DAN cell lines. A producer cell line that can be used for the production of recombinant retrovirus is the producer cell line designated PA317/G1TK1SvNa, which is disclosed in PCT application WO 95/06486.

The retroviral vectors are administered to the host in an amount effective to inhibit, prevent, or destroy the angiogenic properties of VEGF produced by tumor cells, or other pathogenic cells. The host may be a mammalian host, including human and non-human primate hosts. Such administration may be by direct administration of the retroviral vectors to the area of pathogenic cells (for example the tumor itself, or the area of the retina in the case of diabetic retinopathy) of the host, whereby the retroviral vectors transduce the replicating cells. The locus of administration of the retroviral vectors is dependent upon factors which include the nature of the disorder being treated. In general, the retroviral vectors are administered in an amount of at least 10⁴ cfu/dose, and in general, such amount does not exceed 10⁸ cfu/dose. Preferably, the retroviral vectors are administered in an amount of from about 10⁵ cfu/dose to about 10⁷ cfu/dose. The exact dosage is dependent upon a variety of factors, which may include the age, weight, and sex of the patient, the nature of the disorder being treated, and the severity of the disorder being treated.

The retroviral vectors also may be administered in conjunction with an acceptable pharmaceutical carrier, such as, for example, saline solution, protamine sulfate (Elkins-Sinn, Inc., Cherry Hill, N.J.), water, aqueous buffers, such as phosphate buffers and Tris buffers, or Polybrene (Sigma Chemical, St. Louis, Mo.). The selection of a suitable pharmaceutical carrier is deemed to be apparent to those skilled in the art from the teachings contained herein (*infra*).

In one embodiment, the retroviral composition encoding a VEGF binding member with a cellular retention signal is for use by being administered in combination with a chemotherapeutic agent, together which cause inhibition and/or destruction of the growth of the replicating pathogenic cells. In another embodiment, the retroviral composition encoding a VEGF binding member with a cellular retention signal is for use by being administered in combination with an anti-angiogenic agent, together which cause inhibition and/or prevention of angiogenesis.

### Pharmaceutical compositions and administration thereof

Pharmaceutical compositions of the invention can be administered parenterally, topically, orally, or locally, such as by aerosol or transdermally, for prophylactic and/or therapeutic treatment. The pharmaceutical compositions can be administered in a variety of unit dosage forms depending upon the method of administration. For example, unit dosage forms suitable for oral administration include powder, tablets, pills, capsules and lozenges. It is recognized that a nucleic acid encoding the VEGF binding member with an intracellular retention signal, a viral vector containing nucleic acid encoding the VEGF binding member with an intracellular retention signal, etc., when administered orally, must be protected from digestion. This is typically accomplished either by complexing the pharmaceutical composition with a composition to render it resistant to acidic and enzymatic hydrolysis or by packaging the pharmaceutical composition in an appropriately resistant carrier such as a liposome. Means of protecting pharmaceutical compositions (such as those comprising nucleic acid and/or protein) from digestion are well known in the art.

The pharmaceutical compositions of this invention are particularly useful for parenteral administration, such as intravenous administration or administration into a body cavity or lumen of an organ. Alternatively, the pharmaceutical compositions may be administered directly to the site of therapy, such as directly into a tumor, or into the retina as necessary for example in diabetic retinopathy. A variety of aqueous carriers can be used, e.g., buffered saline and the like. These solutions are sterile and generally free of undesirable matter. These compositions may be sterilized by conventional, well known sterilization techniques. The compositions may contain pharmaceutically acceptable auxiliary substances as required to approximate physiological conditions such as pH adjusting and buffering agents, toxicity adjusting agents and the like, for example, sodium acetate, sodium chloride, potassium chloride, calcium chloride, sodium lactate and the like. The concentration of nucleic acid encoding the VEGF binding member with an intracellular retention signal in these formulations can vary widely, and will be selected primarily based on fluid volumes, viscosities, body weight and the like in accordance with the particular mode of administration selected and the patient's needs.

Thus, a typical pharmaceutical composition for intravenous administration would be about 0.1 mg to 10 mg per patient per day. Dosages from 0.1 mg up to about 100 mg per patient per day may be used, particularly when the drug is administered to a secluded site, for example, and not into the bloodstream, such as into a body cavity or into a lumen of an organ. Actual methods for preparing parenterally administrable compositions will be known or apparent to those skilled in the art and are described in more detail in such publications as REMINGTON'S PHARMACEUTICAL SCIENCE, 15th ed., Mack Publishing Company, Easton, Pa. (1980).

The pharmaceutical composition of the present invention may also include pharmaceutically acceptable excipients or auxiliary agents, including, but not limited to, glidants, dissolution agents, surfactants, diluents, binders including low temperature melting binders, disintegrants, solubilizing agents and/or lubricants.

In therapeutic applications, compositions are administered to a patient suffering from a disease, in a cytotoxic amount, an amount sufficient to inhibit/prevent angiogenesis. An amount adequate to accomplish this is defined as a "therapeutically effective dose." Amounts effective for this use will depend upon the severity of the disease and the general state of the patient's health.

Single or multiple administrations of the compositions may be administered depending on the dosage and frequency as required and tolerated by the patient. In any event, the composition should provide a sufficient quantity of the compositions of this invention to effectively treat the patient

Embodiments of the VEGF binding members with cellular retention signals provided herein may be used in combination with other compositions and procedures for the treatment of diseases. For example, but not limited to, a tumor may be treated conventionally with surgery, radiation, chemotherapy, or immunotherapy, combined with VEGF binding members with cellular retention signals and then the VEGF binding members with cellular retention signals may be subsequently administered to the patient to extend the dormancy of micrometastases and to stabilize and inhibit the growth of any residual primary tumor. VEGF binding members with cellular retention signals can also be combined with other anti-angiogenic compounds, or proteins, fragments, antisera, receptor agonists, receptor antagonists of other anti-angiogenic proteins (e.g., angiostatin, endostatin). The compositions may further contain other agents which either enhance the activity of the VEGF binding members with cellular retention signals or compliment its activity or use in treatment, such as chemotherapeutic or radioactive agents. Such additional factors and/or agents may be included in the composition to produce a synergistic effect with protein of the invention, or to minimize side effects.

Although the present invention has been described in some detail by way of illustration and example for purposes of clarity of understanding, it will be obvious that certain changes and modifications may be practiced within the scope of the appended claims.

### Expression of angiogenic factors

Vascular endothelial growth factor/vascular permeability factor (VEGF/VPF) is one angiogenic factor frequently utilized by tumors and other tissues to switch on their angiogenic phenotypes. In fact, nearly all tumors express VEGF at high levels. Recent studies show that VEGF-stimulated blood vessels are not only important for primary tumor growth but also for metastasis. In addition to pathological angiogenesis, VEGF is a factor that contributes to formation of the circulatory system by stimulating vasculogenesis and angiogenesis during embryonic development VEGF-induced vascularization is important for a variety of physiological processes including organ formation, female reproduction, and wound healing. VEGF is the prototype of a growth factor family that contains structurally related members, including placenta growth factor (PLGF), VEGF-B, VEGF-C, and VEGF-D. The angiogenic signals triggered by any of the VEGF members can be mediated by activation of two structurally related homologous tyrosine kinase receptors, VEGFR-1 and VEGFR2, both of which are expressed almost exclusively on endothelial cells. VEGF binds to VEGFR-1 and VEGFR-2, and induces vasculogenesis, angiogenesis and vascular permeability, whereas PLGF and VEGF-B only bind to VEGFR-1 with unknown physiological and pathological functions. However, some recent studies suggest that PLGF-2 may contribute to differentiation of endothelial precursor cells.

In addition to VEGFR-1 and VEGFR-2, a lymphatic endothelial cell specific tyrosine kinase receptor, VEGFR-3, has been identified. VEGF-C and VEGF-D interact with both VEGFR-2 and VEGFR-3, and can induce both blood angiogenesis and lymphangiogenesis. VEGFR-2 is occasionally expressed in lymphatic endothelium. Accumulating evidence points to the fact that activation of VEGFR-2 can trigger angiogenic signals for blood vessel growth, whereas activation of VEGFR-3 can induce lymphangiogenesis.

The function of VEGFR-1 is poorly understood. Some studies suggest a direct role in transducing angiogenic signals, whereas others report that VEGFR-1 may act as a decoy receptor for VEGF/VEGFR-2 signalling. Very recently, VEGFR-1 has been found to play a role in recruiting stem cell-differentiated endothelial cells into newly formed blood vessels.

Similar to VEGF, alternative splicing of human PLGF transcripts generates at least three isoforms of the mature PLGF protein, PLGF-1 (SEQ ID NO: 2), PLGF-2 (SEQ ID NO: 5) and PLGF-3. Like several other growth factors, all members of VEGF naturally exist as dimeric proteins in order to interact with their specific receptors. Based on their expression patterns, homodimers and heterodimers with distinct biological activities are formed. PLGF-1 forms heterodimers with VEGF intracellularly. PLGF-1/VEGF heterodimers are naturally present in tissues when both factors are produced in the same cell.

Knowing the molecular mechanisms and signaling pathways, various approaches have been developed as therapeutic strategies in order to block VEGF function. Consequently, anti-VEGF reagents, including VEGF neutralizing antibodies, VEGF antisense oligonucleotides, soluble VEGF receptors, anti-VEGF receptor antibodies and intracellular signalling inhibitors have produced promising anti-tumor effects in animal models. Encouraged by these pre-clinical studies, about 10 VEGF antagonists are currently in human cancer trials. However, early clinical evaluation of these anti-VEGF compounds has presented some unexpected results. For example, a humanized anti-VEGF antibody and several small anti-VEGF molecules have produced little, if any, beneficial effects. These disappointments have raised several important issues, including an urgent need to improve current anti-VEGF therapeutic strategies. The approaches used today are mainly based on development and administration of functional recombinant protein antagonists that either neutralizes the extracellular VEGF function or block VEGF signalling in target cells. However, none of these strategies are aimed for blockage of VEGF secretion in tumor cells.

The disadvantages with current therapeutic strategies are many, including difficulties in manufacturing active recombinant protein, high dose requirements, high costs for both manufactures and consumers, and the probable need for lifetime treatment of the patient. Due to the relative short half-lives, recombinant proteins must be repeatedly administrated through injection, from once to several times daily. Gene therapy as an alternative approach that can bypass several of the disadvantages with protein therapy. Aspects of the present invention provide alternative approaches for anti-VEGF gene therapy by blocking its secretion from cells, such as from tumor cells.

### RESULTS

### Generation of retroviral vectors containing PLGF-KDEL or VEGF-KDEL

Both PLGF-1 and VEGF can be released *via* the classical secretory pathway and their functional dimers can be formed in the ER. For construction of ER-retained PLGF-1 or VEGF, the C-terminus of human or VEGF was fused with KDEL (SEQ ID NO: 7), a mammalian ER-retention signal. Correct fusion of the gene constructs was confirmed by sequence analysis. The fusion gene products were cloned separately into a retroviral vector containing green fluorescent protein (GFP) as a marker, and recombinant retroviruses were used to transduce a well-characterized murine Lewis lung carcinoma (LLC) cell line, the in *vivo* growth of which is VEGF dependent. The presence of hPLGF-1 (SEQ ID NO: 1) and hVEGF cDNAs was confirmed by Southern blot analysis and GFP positive cells were sorted by FACS analysis.

### Blockage of VEGF secretion in tumor cells

To quantify the amounts of intracellular and extracellular dimeric molecules, a sensitive sandwich ELISA assay was used to analyze cell lysates and conditioned media from transduced and non-transduced LLC cell lines. Specific antibodies were used against each factor, either two antibodies against the same factor but raised in different species (homodimers) or two antibodies against different factors (heterodimers). As expected, a high level of mVEGF homodimers was detected in conditioned media from controls, *wt* LLC and vector-transduced LLC cells (Table 1). The majority of mPLGF-1 produced by *wt* and vector-transduced LLC cells were involved in heterodimerization with mVEGF, suggesting that mPLGF-1 preferentially formed heterodimers with mVEGF, rather than mPLGF-1/mPLGF-1 homodimers. Overexpression of hVEGF in these cells resulted in sufficient secretion of heterodimers of hVEGF/mVEGF molecules (3779 pg/ml) in addition to hVEGF/hVEGF homodimers (41880 pg/ml).

In contrast, transduction of LLC cells with hVEGF-KDEL effectively prevented VEGF secretion, only a minor part of hVEGF/mVEGF (268 pg/ml) and hVEGF/hVEGF (628 pg/ml) were present in conditioned medium compared to the large portion retained intracellularly (1578 pg/ml and 2624 pg/ml, respectively). This demonstrates the functional consequence of the KDEL (SEQ ID NO: 7) being sufficiently retained in the ER. Consistent with our previous report, virtually all mVEGF molecules were present as hPLGF-1/mVEGF heterodimers in the conditioned medium of PLGF-1-overexpressing LLC cells (5581 pg/ml) (Table 1). The preferential formation of hPLGF-1/mVEGF heterodimers in these tumor cells resulted in an efficient depletion of secreted mVEGF homodimers (Table 1). Remarkably, gene delivery of hPLGP-1-KDEL in LLC cells not only enforced nearly all mVEGF molecules to form hPLGF-1/VEGF heterodimers but also prevented the secretion of hPLGF-1/hPLGF-1 homodimers and hPLGF-1/mVEGF heterodimers. The majority of each kind of hetero- and homodimer was present intracellularly, and only minor portions were present in the conditioned medium.

### Depletion of endothelial stimulatory activity released by tumor cells

To monitor the VEGF-mediated endothelial activity, the endothelial chemotactic activity was determined using conditioned media from various transduced tumor cells using a modified Boyden chemotaxis assay. The VEGFR-1- and VEGFR-2- expressing porcine aortic endothelial (PAE) cells have previously been used to detect VEGF activity. When purified recombinant dimeric growth factors were analyzed in this assay, only VEGF homodimers could significantly induce the motility of VEGFR-2/PAE cells (Fig. 1d). Neither PLGF-1 homodimers nor PLGF-1/VEGF heterodimers induced increased cell motility over the background level. As expected, conditioned media from non-transduced or vector-transduced LLC cells significantly stimulated VEGFR-2/PAE cell migration (Fig. 1e and f). However, overexpression of PLGF-1 (SEQ ID NO: 2) or PLGF-1-KDEL (SEQ ID NO: 3) dramatically blocked LLC cell-produced VEGF activity (P<0.001) (Fig. 1e). High expression levels of hVEGF enhanced the chemotactic activity produced by LLC cells (Fig. If). In contrast, overexpression of VEGF-KDEL in these tumor cells drastically abolished VEGFR2/PAE cell migration in comparison to the controls (P<0.001) (Fig. If). None of the recombinant factors or conditioned media induced VEGFR-1/PAE cell motility (Fig. la-c). Similar results were obtained with primary HUVE cells (data not shown). Consistent with the ELISA quantification analysis, these results indicate that the KDEL (SEQ ID NO: 7) - coupled to PLGF-1 (SEQ ID NO: 2) or VEGF sufficiently block the endogenous mouse VEGF activity.

In addition to chemotaxis, endothelial cell morphological changes of VEGFR-1 or VEGFR-2-expressing PAE cells was assayed as independent criteria for evaluation of tumor cell-released VEGF activity. Addition of recombinant hVEGF homodimers at the concentration of SO ng/ml to VEGFR-2/PAE cells induced a spindle-like cell shape with reorganization of actin fibers (Fig. lh), a feature that both PLGF-1 homodimers and PLGF-1/VEGF heterodimers fails to do (Fig. 1i and j). Incubation with conditioned media from *wt* LLC or vector-transfected LLC cells resulted in an elongated spindle-like cell shape in VEGFR-2PAE cells (Fig. 1k and 1), similar to that induced by rhVEGF. Overexpression of hVEGF in LLC cells led to remarkable cell shape changes and actin reorganization of VEGFR-2 expressing PAE cells (Fig. 1o). In contrast, overexpression of hPLGF-1 completely abrogated the morphological changes elicited by VEGF. Consistent with both the ELISA quantitative assay and the chemotaxis analysis, this indicates that a majority of them VEGF molecules participated in the formation of heterodimers with hPLGF-1 (Fig. 1m). Similarly, the VEGF-induced cell shape changes were completely blocked by expression of either PLGF-1-KDEL (SEQ ID NO: 3) or VEGF-KDEL in these tumor cells (Fig. In and p). Again, conditioned media from either cell line failed to induce a similar change in endothelial morphology of VEGFR-1/PAE cells (data not shown).

### Suppression of tumor growth

Although PLGF-1 and VEGF are considered as specific growth factors acting on blood vessel endothelial cells, overexpression and retention of these factors in the ER compartment might affect tumor cell growth. To exclude this possibility, the growth rate of PLGF-1-KDEL (SEQ ID NO: 3) and VEGF-KDEL LLC cells were compared with those of control cells. Transduction of PLGF-1-KDEL (SEQ ID NO: 3) or PLGF-1 (SEQ ID NO: 2) into LLC cells did not alter the growth rates in culture as compared with *wt* LLC and vector-transduced LLC cells, indicating that accumulation of PLGF-1 in the ER compartment did not affect tumor cell growth *in vitro* (Fig. 2a). Similarly, VEGF-KDEL-transduced LLC cells did not demonstrate an altered growth rate *in vitro* when compared to either VEGF overexpressing cells or the two control cell lines (Fig. 3a). The LLC tumor is one of the most aggressive and rapidly growing murine tumors *in vivo.* For the controls, visible tumors were readily detectable 5 days after implantation and grew to the size of the Swedish ethical limit (1500 mm³) within 2 weeks after implantation (Figs. 2c and 3c). Consistent with our recent findings in a murine T241 fibrosarcoma model⁵, expression of hPLGF-1 in LLC remarkably delayed tumor growth and visible tumors were only detectable by day 10 after implantation (Fig. 2c). At day 14 after tumor implantation, approximately 90% inhibition of tumor growth was scored in hPLGF-1-expressing tumors as compared with *wt-* and vector-tranduced tumors (Fig. 2b and c). The tumors remained at a similar small average size, less than 200 mm³, by day 16 after implantation (Fig. 2d).

At day 14 after implantation, hPLGF-1-KDEL-LLC cells only gave rise to barely detectable sizes of tumors, 40 mm (Fig. 2b-d). These tumors remained at similar small sizes for the next three weeks during prolongation of the experiments (Fig. 2d). While hPLGF-1-LLC tumors continued to grow to an average size larger than 600 mm 3-weeks after implantation, hPLGF-1-KDEL-LLC tumors only reached an average size smaller than 100 mm (Fig. 2d). Thus, the measured tumor volumes of hPLGF-1-KDEL-LLC and hPLGF-1-LLC were significantly different (*p*<0.001). The markedly delayed growth rates of hPLGF-1-KDEL-LLC tumors suggest that these tumor cells produce relatively dormant tumors *in vivo,* whereas hPLGF-1-LLC tumors were unable to induce tumor dormancy, and only delayed the angiogenic switch.

To further study if fusion of the KDEL (SEQ ID NO: 7) sequence to VEGF could also inhibit tumor growth, hVEGF-KDEL-LLC cells were implanted into C57B1/6 mice. Although *wt* and vector-transfected LLC cells produced VEGF at high levels, overexpression of hVEGF in these cells could further accelerate tumor growth. After only 10 days the hVEGF-LLC tumors had reached an average size of 1400 mm (close to the Swedish ethical limit) (Fig. 3c and e), whereas both *wt*- and vector-LLC cells needed 14 days to produce a similar size of tumors (Fig. 3b and d). Mice carrying hVEGF-LLC tumors were sacrificed at day 10-post implantation, and at that time 90% inhibition was detected in hVEGF-KDEL-LLC tumors in comparison to hVEGF-LLC tumors (Fig. 3c and e). In contrast to hVEGF-LLC, implantation of hVEGF-KDEL-LLC cells produced approximately 50% inhibition of tumor growth at day 14 when compared with *wt* and vector tumors (Fig. 3b and d). These *in vivo* tumor growth differences were not due to altered tumor cell growth rates as all transduced and non-transduced tumor cells grew at a similar rate *in vitro* (Figs. 2a and 3a).

### Suppression of VEGF-induced tumor neovascularization

To study tumor neovascularization, immunohistochemical analysis was performed using an anti-CD31 antibody. Human PLGF-1-KDEL-LLC tumors had significantly reduced neovascularization as compared to *wt*- or vector-transduced-LLC tumors (Fig. 4a, b, f and g). Consistent with the early findings in a murine fibrosarcoma model, overexpression of PLGF-1 in LLC resulted in significant inhibition of LLC tumor neovascularization (Fig. 4e and g). However, hPLGF-1-KDEL was significantly more potent than PLGF-1 in blocking tumor neovascularization (Fig. 4f and g). Transduction of LLC with hVEGF-KDEL also dramatically blocked tumor neovascularization. In contrast to hPLGF-1(SEQ ID NO: 2), hPLGF-1-KDEL (SEQ ID NO: 3) and hVEGF-KDEL, transduction of LLC with hVEGF alone remarkably increased tumor neovascularization (Fig. 4c and g), with an average of more than 350 microvessels per optical field (x10).

Confocal analysis of tumor vasculatures revealed that *wt* and vector-transduced tumors contained high numbers of vessels with high densities of capillary sprouts (Fig. 4h and i). Interestingly, extremely high numbers of capillaries or microvessels, that were likely to fuse into primitive vascular plexuses, were found in hVEGF-LLC tumors (Fig. 4j). This type of vascular structures appeared to be leaky and hemorrhagic because autopsy examination of tumor tissues demonstrated that hVEGF-LLC tumors consisted of large internal volumes of hemorrhagic tissue fluids. In contrast, transduction of LLC tumor cells with hVEGF-KDEL blocked capillary sprout formation and resulted in formation of vascular architectures lacking the usual vascular branches (Fig. 4k). Remarkably, overexpression of hPLGF-1-KDEL (SEQ ID NO: 3) in LLC tumors led to not only a drastic reduction of vessel numbers but also a nearly complete depletion of microcapillaries (Fig. 4m). Similarly, PLGF-1-LLC tumors lacked vascular sprouts (Fig. 41). These data demonstrate that overexpression of ER-retained hPLGP-1-KDEL (SEQ ID NO: 3) or hVEGF-KDEL proteins in mouse tumors sufficiently blocks mouse VEGF secretion and tumor neovascularization.

### Induction of tumor cell apoptosis

The growth of blood vessels into tumors not only supplies nutrients and O₂ but can also provide survival factors for tumor cells. Therefore, suppression of tumor angiogenesis might influence the rate of tumor cell apoptosis. To assess tumor cells apoptosis, a TUNNEL staining was carried out. Approximately, an average of 8 apoptotic cells/optical field (40x) were found in the fast growing *wt* and vector-transduced tumors (Fig. 5a, b and g). Overexpression of hVEGF significantly reduced the number of apoptotic tumor cells (4 apoptotic cells/field, P<0.05), suggesting that hVEGF-induced vessels were able to supply additional survival factors and thereby prevent apoptosis of tumor cells (Fig. 5c and g). However, transduction of hVEGF-KDEL in LLC tumor cells resulted in a significant increase of apoptosis (17 apoptotic cells/field, P<0.001) (Fig. 5d and g). In the hPLGF-1-KDEL-(SEQ ID NO: 3), and hPLGF-1 (SEQ ID NO: 2)-transduced LLC tumors, the increase in number of apoptotic cells was also found to be highly significant (19 and 22 apoptotic cells/field, respectively, *P*<0.001) (Fig. 5e, f and g). According to our previous results, even a small increase of tumor cell apoptosis could have great impact on tumor volume because the turn-over rate of tumor cells is relatively fast. These data indicate that in hVEGF-KDEL-and hPLGF-1-KDEL-(SEQ ID NO: 3) transduced tumors a massive number of tumor cells undergo apoptosis due to insufficient blood supply.

### Discussion

VEGF is believed to be one factor that switches on an angiogenic phenotype in most if not all tumors. The role of VEGF in regulation of diseases is not only limited to cancer. Other angiogenesis dependent diseases, including diabetic retinopathy, age-related macular degeneration, arteriosclerosis-related ischemic heart disease, and stroke are related to VEGF as well. Thus, development of VEGF antagonists has become one of the central focuses in current antiangiogenic therapy for the treatment of cancer and other common diseases. These antagonists target VEGF ligands, receptors and intracellular signaling components. In animal disease models, successful delivery of most VEGF antagonists produces remarkable effects in blocking pathological progression, and improving disease conditions. For example, VEGF neutralizing antibodies potently block tumor growth in mice. Promising results from animal studies have stimulated the enthusiasm to test these compounds in the treatment of human diseases. In fact, more than 10 different VEGF antagonists have entered into clinical trials for treatment of human cancers.

As most anti-VEGF reagents inhibit the extracellular VEGF functions, it is likely that these VEGF antagonists may not completely block VEGF activity. Further, these approaches may suffer several potential problems in clinical trials, including: 1) requirement of frequent injections of anti-VEGF recombinant proteins or chemical compounds in order to maintain steady-state levels in the blood; 2) some of the antagonists, such as soluble VEGF receptors, have extremely short half-lives in the circulation, these molecules can be either sequestered in the extracellular matrix compartment by binding to heparin-like structures or quickly cleared from the body; 3) as protein molecules, some of the VEGF antagonists can be easily destroyed by proteases in the body; 4) relatively large doses of VEGF antagonists have to be delivered in order to produce beneficial effects; 5) as several isoforms of VEGF can be generated by alternative RNA-splicing, not all antagonists may effectively block biological functions of all variants of VEGF; 6) VEGF and VEGF receptors may interact with other proteins in the body and thereby obtain a changed conformation, thus, anti-VEGF antibodies may not recognize the original epitopes; and 7) the long term, if not life-span, administration of VEGF antagonists treatment require high costs for both manufacturers and for patients. All these potential problems highlight the need to optimize the anti-VEGF approaches.

Aspects of the present invention provide therapeutic approaches to prevent VEGF secretion from tumor cells. As tumor cells lack high affinity VEGF receptors, sequestration of VEGF as an intracellular protein may not result in activation of "intracrine" signaling pathways. Consistent with this principle, an embodiment of the present invention revealed overexpression of an intracellular VEGF did not alter tumor cell growth rates *in vitro.* In order to prevent VEGF secretion, an intracellular retention signal, KDEL (SEQ ID NO: 7), a four-amino-acid peptide that retains secretory proteins in the ER, was fused to the C-terminus of PLGF-1 (SEQ ID NO: 3). One principle applied to this approach is to use PLGF-1 as bait, which forms biologically inactive heterodimers with VEGF. Overexpression of hPLGF-1-KDEL (SEQ ID NO: 3) in tumor cells forces the majority, if not all, endogenous VEGF monomers to form heterodimers. Thus, this strategy nearly completely inhibits VEGF secretion by tumor cells.

In addition to heterodimers, most PLGF-1 homodimers were retained in the ER compartment without further secretion. Prevention of PLGF-1 homodimer secretion is an important step to further suppress VEGF function. VEGF has a higher binding affinity for VEGFR-1 compared to VEGFR-2 Excessive amounts of extracellular PLGF-1 could compete with VEGF for binding to the VEGFR-1 receptor, a possible decoy receptor. Thus, prevention of PLGF-1 homodimer secretion will reduce the availability of VEGF to interact with VEGFR-2, the receptor that transduces both angiogenic and vascular leakage signals. Prevention of PLGF-1/VEGF heterodimer secretion may further inhibit angiogenic activity as the heterodimers may have some unknown angiogenic properties. As many tumors overexpress PLGF-1 and PLGF-2, blockage of endogenous PLGF secretion by hPLGF-1-KDEL (SEQ ID NO: 3) could further reduce VEGF-induced angiogenesis and tumor growth. Thus, the various aspects of the invention disclosed herein block VEGF at two levels, both intracellularly and extracellularly. As expected, overproduction of hPLGF-1-KDEL (SEQ ID NO: 3) exhibits more potent anti-tumor activity than native PLGF-1.

Although transduction of hVEGF into tumor cells further potentiates tumor angiogenesis and tumor growth, overexpression of hVEGF-KDEL potently suppresses tumor growth as compared to control tumors. These data indicate that coupling of the KDEL (SEQ ID NO: 7) sequence onto the hVEGF sequence, therein forming hVEGF-KDEL, sufficiently blocks endogenous mouse VEGF secretion and antagonizes its activity. Thus, aspects of the present invention provide an efficacious anti-angiogenic therapeutic approach by inhibiting and/or preventing the secretion of VEGF. Embodiments of the invention can be practiced either alone or in combinations with other anti-VEGF methods, to provide efficacious treatments for human cancer and other angiogenesis-dependent diseases.

### EXPERIMENTAL PROCEDURES

### Animals

Female 6-7-wk-old C57B1/6 mice were acclimated and caged in groups of six or less. Animals were anaesthetized by an injection of a mixture of dormicum and hypnorm (1:1) before all procedures and sacrificed by a lethal dose of CO₂. All animal studies were reviewed and approved by the animal care and use committee of the Stockholm Animal Board.

### Generation and purification of PLGF-1/VEGF₁₆₅heterodimers

Recombinant human PLGF-1 (SEQ ID NO: 1)and VEGF₁ monomers were expressed in *E. Coli* as previously described⁴⁴. An equimolar of mixture of PLGF-1-and VEGF₁ homodimers, at a total protein concentration of 0.5 mg/ml, was incubated in reducing buffer (20 mM Tris-HCl, pH 8.0, 6 M guanidine-HC1, and 10 mM DTT) at 4°C over night The following day the protein solution was dialyzed against 10 volumes of refolding buffer (2 M urea, 20 mM Tris-HC1, pH 8.0, 2 mM GSH (glutathione-SH) and 0.5 mM GSSG (glutathione-S-S-glutathione)). Using this refolding protocol, a mixture of homodimeric PLGF-1 and VEGF₁ as well as heterodimeric PLGF-1/VEGF was generated.

The homodimeric and the heterodimeric proteins were separated by affinity chromatography using a goat polyclonal anti-hVEGF affinity column and a polyclonal goat anti-hPLGF affinity column. The protein solution, previously dialyzed against PBS, was initially applied at a flow rate of 2 ml/min onto the anti-hVEGF-affinity column pre-equilibrated with PBS. The column was then washed at the same flow rate with PBS until the absorbance reading at 280 nm reached base-line level. VEGF homodimers and PLGF-1/VEGF heterodimers, but not PLGF homodimers, were retained by the column and eluted with 0.1 M sodium citrate, pH 2.5,0.3 M NaCl (elution buffer). The dimers eluted from the anti-VEGF column were instantly neutralized with 2 M Tris buffer, pH 8, and subsequently dialyzed against 20 volumes of PBS at 4°C for 4 h. After dialysis the protein sample was applied to a PBS pre-equilibrated anti-PLGF affinity column using the same conditions. Only heterodimers were retained and eluted from the column using the same elution buffer. Purified hPLGF-1, hVEGF, and hPLGF-1/hVEGF proteins were finally dialyzed against PBS and analyzed by SDS-PAGE under both reducing and non-reducing conditions, followed by measurement of protein concentrations.

### Retroviral vector design and tumor cell transduction

Complementary cDNAs coding for human PLGF-1(SEQ ID NO: 1) ₁₂₉, PLGF-1₁₂₉-KDEL, VEGF₁₆₅, and VEGF₁₆₅-KDEL were cloned into the Murine Stem Cell Virus (MSCV) vector (kindly provided by Dr. R. Hawley at the Holland Laboratory, Rockville, MD) containing GFP. Transfection of retroviral constructs into 293T cells along with expression plasmids encoding ecotropic gag/pol and the Vesicular Stomatitis Virus-Glycoprotein (VSV-G) envelope protein using a classical CaPO₄ transfection method generated retroviral supernatants. Murine LLC cells grown in log phase were exposed to filtered viral supernatants in the presence of 8 µg/ml of protamine sulfate on RetronectinTM (Biowhittaker, East Rutherford, NJ) coated culture dishes for 6 hours on two consecutive days. GFP positive cells were sorted using a FACStar+ (Becton Dickinson, San Jose, CA) equipped with a 5-W argon and 30-mW neon laser. PCR and Southern blot analyses were performed using standard methods.

### Tumor cell proliferation assay

Vector-, hPLGF-1-(SEQ ID NO: 2), hPLGF-1-KDEL (SEQ ID NO: 3)-, hVEGF- and hVEGF-KDELtransduced, as well as *wt*, LLC cells were seeded at a density of 1 x 10⁴ cells/well in 24-well-plates in DME medium supplemented with 10% FCS and incubated at 37°C. Cells were trypsinized, resuspended in Isoton II solution (Beckman Coulter, Sweden) and counted in a Coulter Counter at various time points. Triplicates were used for each sample, and all experiments were performed three times.

### Cell Shape Assay and Actin Staining

PAE cells expressing either VEGFR-1 or VEGFR-2 were grown on coverslips in 12-well plates, in Ham's F12 medium supplemented with 10% FCS as previously described. At a confluency of about 40-60%, the medium was replaced with fresh Ham's F12 medium containing only 2% FCS and 50 ng/ml of either recombinant factors (hVEGF, hPLGF-1 or hPLGF-1/hVEGF) or 25% (v/v) of conditioned media from LLC cell lines, including *wt*, vector, hPLGF-1, hPLGF-1-KDEL, hVEGF or hVEGF-1-KDEL. Non-treated cells served as a negative control. After incubation for 16 h, cells were fixed with 3% paraformaldehyde (PFA) in PBS (pH 7.5) for 30 min., rinsed three times with PBS, and permeabilized with 0.5% Triton X-100 in PBS for 15 min. The cells were then repeatedly washed with PBS and stained for 30 min with 1 µg/ml of TRITC-phalloidin (Sigma) diluted in PBS. After washing with PBS, the coverslips were mounted in a mixture of glycerol and PBS (9:1). Cells were examined in a combined light and fluorescence microscope and spindle-like cells were counted in 5 optical fields (20x). Data represents mean % (±SEM).

### Chemotaxis Assay

The motility responses of VEGFR-1/PAE and VEGFR-2/PAE cells to various recombinant growth factors and LLC conditioned media were assayed using a modified Boyden chamber technique previously described. Briefly, the ability of VEGFR-expressing PAE cells to migrate through a micropore nitrocellulose filter (8 µm thick, 8 µm pores) was measured as a criterion for chemotactic stimuli. To the lower chambers serum-free medium supplemented with 0.2% BSA and 50ng/ml of either recombinant factors (hVEGF, hPLGF-1 or hPLGF-1/hVEGF) or 25% (v/v) of conditioned media from different retro-virally transduced cells was added. Non-treated cells served as a negative control. Cells were trypsinized and resuspended in serum-free medium (0.2% BSA) at a concentration of 0.8 x 10⁶ cells/ml, and to each well in the upper chamber 40,000 cells were added. After 4 h incubation at 37°C, the Boyden chamber was disassembled and cells attached to the filter were fixed in methanol and stained with Giemsa solution. Quadruplicates of each sample were used, and all experiments were performed three times. The cells that had migrated through the filter were counted using a light microscope, and plotted as numbers of migrating cells per optic field (32x)

### ELISA assay

All sandwich ELISAs were performed using the Quantikine ELISA system (R&D Systems) according to the manufacturer's instruction. Briefly, standard mouse (m)VEGF and samples were added into a 96-well microplate pre-coated with an affinity purified polyclonal antibody specific for mVEGF. Homodimers containing mVEGF were detected by an enzyme-linked polyclonal antibody specific for mVEGF. Similarly, homodimers of mPLGF-1, hVEGF, and hPLGF-1 were measured using the Quantikine M mPLGF-1 ELISA kit, Quantikine hVEGF ELISA kit, and Quantikine hPLGF ELISA kit, respectively. These three kits contain specific monoclonal antibodies as captures.

The heterodimers were measured with cross-matching capture and detection antibodies using the same ELISA kits mentioned above. For mVEGF/mPLGF-1 heterodimers, samples were added onto microplates pre-coated with anti-mVEGF. Enzyme-linked anti-mPLGF-1 was then used to detect the mVEGF/mPLGF-1 heterodimer. To calibrate the assay, a recombinant mPLGF-1 standard was analyzed simultaneously on the PLGF-1 plate. No cross reactivity was observed from the homodimers. Likewise, mVEGF/hVEGF heterodimers were measured using microplates pre-coated with anti-hVEGF antibodies, and anti-mVEGF conjugates for detection of heterodimers. Recombinant mVEGF standards analyzed on the mVEGF plates were used for calibration. Approximately 1-2% of cross reactivity was observed from each homodimer, and the results were corrected accordingly. mVEGF/hPLGF-1 heterodimers were measured using microplates pre-coated with anti-mVEGF antibodies, and an anti-hPLGF-1 conjugate for detection. Recombinant hPLGF-1 standards analyzed on the hPLGF-1 plates were used for calibration. Approximately 3% of cross reactivity were observed from the hPLGF-1 homodimer, the results were corrected accordingly.

### Tumor studies in mice

Wild type-LLC, vector transfected-LLC, and LLC cells expressing hPLGF-1, hPLGF-1-KDEL, hVEGF or hVEGF-KDEL were used for tumor implantation studies in 6-7-wk-old syngeneic C57B1/6 mice. Approximately 1 x 10⁶ tumor cells were implanted subcutaneously on the back of each mouse. Six mice were used in both treated and control groups. Primary tumors were measured using digital calipers on the days indicated. Tumor volumes were calculated according to the formula: width² x length x 0.52, and when the Swedish ethical limit (1500 mm³) was reached the tumors were removed.

### Histology

Tumors were surgically removed when they reached the Swedish ethical limit. For quantification of tumor vessels immunohistochemistry was carried out using an anti-CD31 antibody. Tumor tissues were fixed in 3% PFA, dehydrated and embedded in paraffin. Samples were sectioned (6 *µ*m thickness) and treated with 20 *µ*gml⁻¹ proteinase K (LifeTechnologies). The background level of peroxidase activity was quenched with 0.3% H₂O₂, and endogenous biotin and avidin activity was blocked using an Avidin/Biotin Blocking kit (Vector Laboratories Inc., Burlingame, CA). Sections were immunostained using a biotinylated monoclonal antibody against CD31 (Pharmingen, San Diego, CA), followed by horseradish peroxidase (HRP) conjugated streptavidin (SA). The tyramide signal amplification (TSA) kit (NEN Life Science, Boston, MA) was used to enhance staining signals. Peroxidase activity was developed using diaminobenzidine (DAB, Vector Laboratories Inc., Burlingame, CA). The sections were photographed and blood vessels were counted under a light microscope in 6 optical fields (20x). Data represents mean % (±SEM).

### Confocal microscopy analysis

To directly visualize tumor vascularization, whole mount staining and confocal microscopy analysis were performed. Tumors were dissected into thin tissue slices and fixed in 3% PFA overnight. Antibody epitopes within the tissue were exposed through proteinase K (20 *µ*gml⁻¹) digestion and methanol permeabilization. Endogenous biotin and avidin activity was blocked before staining with a biotinylated rat anti-mouse monoclonal antibody against CD31 (Pharmingen, San Diego, CA). Blood vessels were detected with SA-Cy3 (Jackon ImmunoResearch Laboratories Inc.), and visualized using confocal microscopy (Zeiss Confocal LSM510 Microscope). By scanning 16 thin sections (5-6 *µ*m distance) of each sample, three-dimensional images of each tissue sample were assembled.

### TUNEL staining

For detection of apoptotic cells in the tumors a TUNEL staining was performed. Tumor tissues were fixed with 3% paraformaldehyde, dehydrated and embedded in paraffin. Dewaxed and rehydrated tissue sections (5 *µ*m thickness) were TUNEL stained according to a standard, but modified, fluorescein *in situ* Death Detection Kit (Amersham). Briefly, the tissues were treated with 20 *µ*gml⁻¹ proteinase K (LifeTechnologies), and the background level of peroxidase was blocked with 3% H₂O₂ in methanol. A TUNEL reaction mixture was added to sections and incubated in a humid atmosphere at 37 °C for 1 h, followed by counter staining with Hoescht 33258 (500 ngml⁻¹). The sections were photographed and apoptotic cells were counted under a fluorescence microscope in 10 optical fields (40x), Data represents mean determinants (± SEM).

### Statistical analysis

Statistical analysis was carried out using standard Student's two-tailed *t*-test in Microsoft Excel. *P*-values below 0.05 (*) and < 0.001 (***) were deemed as significant and highly significant, respectfully.

## Claims

1. An isolated nucleic acid comprising a cDNA sequence identical to a nucleic acid encoding a VEGF binding member, with an intracellular retention signal; wherein the VEGF binding member is selected from PLGF, VEGF-B, VEGF receptor (VEGFR) 1 (VEGFR-1), VEGFR-2, neuropilin-1 and neuropilin-2.

2. The nucleic acid according to claim 1, wherein the VEGF binding member is selected from PLGF-1 (SEQ ID NO: 2), PLGF-2 (SEQ ID NO: 5), PLGF-3, VEGF-B (SEQ ID NO: 11 or 14), VEGF receptor (VEGFR) 1 (VEGFR-1), VEGFR-2, neuropilin-1 and neuropilin-2.

3. The nucleic acid according to claim 1, wherein the VEGF binding member is selected from PLGF and VEGF-B.

4. The nucleic acid according to claim 1, wherein the VEGF binding member is selected from PLGF-1 (SEQ ID NO: 2), PLGF-2 (SEQ ID NO: 5), PLGF-3, and VEGF-B (SEQ ID NO: 11 or 14).

5. The nucleic acid according to any one of the claims 1-4, wherein the intracellular retention signal is an endoplasmic reticulum retention signal, a Golgi retention signal, an endosome/lysosome retention sequence, a mitochondria targeting sequence, a nucleus targeting sequence, or a peroxisome targeting sequence.

6. The nucleic acid according to claim 5, wherein the intracellular retention signal is an endoplasmic reticulum retention signal according to SEQ ID NO: 7, a Golgi retention signal according to SEQ ID NO: 19, or a lysosome retention sequence according to SEQ ID NO: 16.

7. The nucleic acid according to claim 5, wherein the intracellular retention signal is an endoplasmic reticulum retention signal

8. The nucleic acid according to claim 7, wherein the endoplasmic reticulum retention signal comprises a sequence according to SEQ ID NO: 7.

9. A vector for expressing a VEGF binding member with an intracellular retention signal, selected from a recombinant plasmid and a recombinant viral vector, comprising a nucleic acid according to anyone of the claims 1-8.

10. The vector according to claim 9, wherein the recombinant viral vector is selected from the group consisting of an adenoviral vector, an adeno-associated viral vector, a vaccinia viral vector, a lentiviral vector, a retroviral vector, and a herpes virus vector.

11. A pharmaceutical composition comprising a vector according to claim 9 or claim 10, and a pharmaceutically acceptable carrier.

12. The pharmaceutical composition according to claim 11, for use in the treatment of an angiogenic disease.

13. The pharmaceutical composition according to claim 12, wherein the angiogenic disease is a disease selected from the group of cancers, inflammatory arthritis, diabetic retinopathy, neovascular disease of the eye, arteriovenous malformations, conditions of excessive bleeding, Osier-Webber Syndrome, myocardial angiogenesis, plaque neovascularization, telangiectasia, hemophiliac joints, angiofibroma, wound granulation, and diseases of excessive or abnormal stimulation of endothelial cells.

14. The vector according to claim 9 or claim 10, for use in therapy.

15. The vector according to claim 9 or claim 10, for use in the treatment of an angiogenic disease.

16. The vector according to claim 15, wherein the angiogenic disease is a disease selected from the group of cancers, inflammatory arthritis, diabetic retinopathy, neovascular disease of the eye, arteriovenous malformations, conditions of excessive bleeding, Osier-Webber Syndrome, myocardial angiogenesis, plaque neovascularization, telangiectasia, hemophiliac joints, angiofibroma, wound granulation, and diseases of excessive or abnormal stimulation of endothelial cells.

17. Use of a vector according to claim 9 or claim 10, in the manufacturing of a medicament for the treatment of an angiogenic disease.

## Patentansprüche

1. Isolierte Nucleinsäure, umfassend eine mit einer Nucleinsäure, die ein VEGF bindendes Mitglied kodiert, identische cDNA-Sequenz mit einem intrazellulären Retentionssignal; wobei das VEGF bindende Mitglied aus PLGF, VEGF-B, VEGF-Rezeptor (VEGFR) 1 (VEGFR-1), VEGFR-2, Neuropilin-1 und Neuropilin-2 ausgewählt ist.

2. Nucleinsäure gemäß Anspruch 1, wobei das VEGF bindende Mitglied aus PLGF-1 (SEQ ID NO: 2), PLGF-2 (SEQ ID NO: 5), PLGF-3, VEGF-B (SEQ ID NO: 11 oder 14), VEGF-Rezeptor (VEGFR) 1 (VEGFR-1), VEGFR-2, Neuropilin-1 und Neuropilin-2 ausgewählt ist.

3. Nucleinsäure gemäß Anspruch 1, wobei das VEGF bindende Mitglied aus PLGF und VEGF-B ausgewählt ist.

4. Nucleinsäure gemäß Anspruch 1, wobei das VEGF bindende Mitglied aus PLGF-1 (SEQ ID NO: 2), PLGF-2 (SEQ ID NO: 5), PLGF-3 und VEGF-B (SEQ ID NO: 11 oder 14) ausgewählt ist.

5. Nucleinsäure gemäß einem der Ansprüche 1 - 4, wobei es sich bei dem intrazellulären Retentionssignal um ein Retentionssignal für endoplasmatisches Retikulum, ein Golgi-Retentionssignal, eine Endosom/Lysosom-Retentionssequenz, eine Mitochondrien-Targetingsequenz, eine Nucleus-Targetingsequenz oder eine Peroxisom-Targetingsequenz handelt.

6. Nucleinsäure gemäß Anspruch 5, wobei es sich bei dem intrazellulären Retentionssignal um ein Retentionssignal für endoplasmatisches Retikulum gemäß SEQ ID NO: 7, ein Golgi-Retentionssignal gemäß SEQ ID NO: 19 oder eine Lysosom-Retentionssequenz gemäß SEQ ID NO: 16 handelt.

7. Nucleinsäure gemäß Anspruch 5, wobei es sich bei dem intrazellulären Retentionssignal um ein Retentionssignal für endoplasmatisches Retikulum handelt.

8. Nucleinsäure gemäß Anspruch 7, wobei das Retentionssignal für endoplasmatisches Retikulum eine Sequenz gemäß SEQ ID NO: 7 umfasst.

9. Vektor zum Exprimieren eines VEGF bindenden Mitglieds mit einem intrazellulären Retentionssignal, ausgewählt aus einem rekombinanten Plasmid und einem rekombinanten Virusvektor, umfassend eine Nucleinsäure gemäß einem der Ansprüche 1 - 8.

10. Vektor gemäß Anspruch 9, wobei der rekombinante Virusvektor aus der Gruppe ausgewählt ist, bestehend aus einem Adenovirusvektor, einem Adeno-assoziierten Virusvektor, einem Vacciniavirusvektor, einem Lentivirusvektor, einem Retrovirusvektor und einem Herpesvirusvektor.

11. Pharmazeutische Zusammensetzung, umfassend einen Vektor gemäß Anspruch 9 oder Anspruch 10 und einen pharmazeutisch verträglichen Träger.

12. Pharmazeutische Zusammensetzung gemäß Anspruch 11 zur Verwendung bei der Behandlung einer Angiogenese-Erkrankung.

13. Pharmazeutische Zusammensetzung gemäß Anspruch 12, wobei es sich bei der Angiogenese-Erkrankung um eine Erkrankung handelt, ausgewählt aus der Gruppe von Krebserkrankungen, entzündlicher Arthritis, diabetischer Retinopathie, neovaskulärer Augenerkrankung, arteriovenösen Fehlbildungen, Zuständen exzessiver Blutung, Morbus Osler, Myokard-Angiogenese, Plaque-Neovaskularisierung, Telangiektasie, Blutergelenken, Angiofibrom, Wundgranulation und Erkrankungen exzessiver oder anormaler Endothelzellstimulation.

14. Vektor gemäß Anspruch 9 oder Anspruch 10 zur Verwendung in der Therapie.

15. Vektor gemäß Anspruch 9 oder Anspruch 10 zur Verwendung bei der Behandlung einer Angiogenese-Erkrankung.

16. Vektor gemäß Anspruch 15, wobei es sich bei der Angiogenese-Erkrankung um eine Erkrankung handelt, ausgewählt aus der Gruppe von Krebserkrankungen, entzündlicher Arthritis, diabetischer Retinopathie, neovaskulärer Augenerkrankung, arteriovenösen Fehlbildungen, Zuständen exzessiver Blutung, Morbus Osler, Myokard-Angiogenese, Plaque-Neovaskularisierung, Telangiektasie, Blutergelenken, Angiofibrom, Wundgranulation und Erkrankungen exzessiver oder anormaler Endothelzellstimulation.

17. Verwendung eines Vektors gemäß Anspruch 9 oder Anspruch 10 bei der Herstellung eines Medikaments zur Behandlung einer Angiogenese-Erkrankung.

## Revendications

1. Acide nucléique isolé comprenant une séquence d'ADNc identique à un acide nucléique codant un élément de liaison au VEGF, avec un signal de rétention intracellulaire ; dans lequel l'élément de liaison au VEGF est choisi parmi le PLGF, le VEGF-B, le récepteur du VEGF (VEGFR) 1 (VEGFR-1), le VEGFR-2, la neuropiline-1 et la neuropiline-2.

2. Acide nucléique selon la revendication 1, dans lequel l'élément de liaison au VEGF est choisi parmi le PLGF-1 (SEQ ID NO: 2), le PLGF-2 (SEQ ID NO: 5), le PLGF-3, le VEGF-B (SEQ ID NO: 11 ou 14), le récepteur du VEGF (VEGFR) 1 (VEGFR-1), le VEGFR-2, la neuropiline-1 et la neuropiline-2.

3. Acide nucléique selon la revendication 1, dans lequel l'élément de liaison au VEGF est choisi parmi le PLGF et le VEGF-B.

4. Acide nucléique selon la revendication 1, dans lequel l'élément de liaison au VEGF est choisi parmi le PLGF-1 (SEQ ID NO: 2), le PLGF-2 (SEQ ID NO: 5), le PLGF-3 et le VEGF-B (SEQ ID NO: 11 ou 14).

5. Acide nucléique selon l'une quelconque des revendications 1 à 4, dans lequel le signal de rétention intracellulaire est un signal de rétention dans le réticulum endoplasmique, un signal de rétention dans l'appareil de Golgi, une séquence de rétention dans les endosomes/lysosomes, une séquence de ciblage mitochondriale, une séquence de ciblage nucléaire ou une séquence de ciblage peroxisomale.

6. Acide nucléique selon la revendication 5, dans lequel le signal de rétention intracellulaire est un signal de rétention dans le réticulum endoplasmique selon la SEQ ID NO: 7, un signal de rétention dans l'appareil de Golgi selon la SEQ ID NO: 19 ou une séquence de rétention dans les lysosomes selon la SEQ ID NO: 16.

7. Acide nucléique selon la revendication 5, dans lequel le signal de rétention intracellulaire est un signal de rétention dans le réticulum endoplasmique.

8. Acide nucléique selon la revendication 7, dans lequel le signal de rétention dans le réticulum endoplasmique comprend une séquence selon la SEQ ID NO: 7.

9. Vecteur pour exprimer un élément de liaison au VEGF avec un signal de rétention intracellulaire, choisi parmi un plasmide recombinant et un vecteur viral recombinant, comprenant un acide nucléique selon l'une quelconque des revendications 1 à 8.

10. Vecteur selon la revendication 9, dans lequel le vecteur viral recombinant est choisi dans le groupe constitué par un vecteur adénoviral, un vecteur viral adéno-associé, un vecteur viral de la vaccine, un vecteur lentiviral, un vecteur rétroviral et un vecteur du virus de l'herpès.

11. Composition pharmaceutique comprenant un vecteur selon la revendication 9 ou la revendication 10, et un véhicule pharmaceutiquement acceptable.

12. Composition pharmaceutique selon la revendication 11, pour utilisation dans le traitement d'une maladie angiogénique.

13. Composition pharmaceutique selon la revendication 12, dans laquelle la maladie angiogénique est une maladie choisie dans le groupe des cancers, d'une arthrite inflammatoire, d'une rétinopathie diabétique, d'une maladie néovasculaire de l'oeil, des malformations artérioveineuses, des affections de saignement excessif, d'un syndrome d'Osier-Webber, d'une angiogenèse myocardique, d'une néovascularisation de plaque, d'une télangiectasie, des articulations hémophiliques, d'un angiofibrome, d'une granulation de blessure et des maladies de stimulation excessive ou anormale des cellules endothéliales.

14. Vecteur selon la revendication 9 ou la revendication 10, pour utilisation en thérapie.

15. Vecteur selon la revendication 9 ou la revendication 10, pour utilisation dans le traitement d'une maladie angiogénique.

16. Vecteur selon la revendication 15, dans lequel la maladie angiogénique est une maladie choisie dans le groupe des cancers, d'une arthrite inflammatoire, d'une rétinopathie diabétique, d'une maladie néovasculaire de l'oeil, des malformations artérioveineuses, des affections de saignement excessif, d'un syndrome d'Osier-Webber, d'une angiogenèse myocardique, d'une néovascularisation de plaque, d'une télangiectasie, des articulations hémophiliques, d'un angiofibrome, d'une granulation de blessure et des maladies de stimulation excessive ou anormale des cellules endothéliales.

17. Utilisation d'un vecteur selon la revendication 9 ou la revendication 10, dans la fabrication d'un médicament pour le traitement d'une maladie angiogénique.
